# EUROPÄISCHE PATENTSCHRIFT

(11) **EP 0 581 953 B1**
(45) Veröffentlichungstag und Bekanntmachung des Hinweises auf die Patenterteilung: **27.05.1998**
(21) Anmeldenummer: 93917362.1
(22) Anmeldetag: 04.02.1993
(51) Int. Cl.: C12Q 1/68, B29C 51/00, B01L 7/00, B29C 65/02, B65B 9/04, G05D 23/19

(54) **VERFAHREN ZUR BESTIMMUNG VON $i(IN VITRO) AMPLIFIZIERTEN NUKLEINSÄUREN**
PROCESS FOR DETERMINING $i(IN VITRO) AMPLIFIED NUCLEIC ACIDS
PROCEDE DE DETERMINATION D'ACIDES NUCLEIQUES AMPLIFIES $i(IN VITRO)

(30) Priorität: 05.02.1992 DE 4203178; 09.10.1992 DE 4234086
(43) Veröffentlichungstag der Anmeldung: 09.02.1994
(73) Patentinhaber: Evotec BioSystems GmbH, D-22529 Hamburg (DE)
(72) Erfinder: HENCO, Karsten, D-4006 Erkrath 2 (DE); EIGEN, Manfred, D-3400 Göttingen (DE); RIESNER, Detlev, D-41541 Dormagen (DE)
(74) Vertreter: Meyers, Hans-Wilhelm, Dr.Dipl.-Chem.
(86) Internationale Anmeldenummer: EP9300254
(87) Internationale Veröffentlichungsnummer: WO9316194

(56) Entgegenhaltungen:
- EP-A- 0 381 501
- WO-A-90/01563
- WO-A-90/05023
- WO-A-90/15881
- WO-A-91/02815
- WO-A-91/02817
- WO-A-92/01533
- NUCLEIC ACIDS RESEARCH Bd. 18, Nr. 22, 1990, ARLINGTON, VIRGINIA US Seiten 6733 - 6734 K. HENCO ET AL. 'Quantitqtive PCR: the determination of template copy numbers by temperature gradient gel electrophoresis (TGGE)' in der Anmeldung erw hnt
- ANN. REV. PHYS. CHEM. Bd. 39, 1988, Seiten 291 - 315 J. E. HEARST 'A photochemical investigation of the dynamics of the oligonucleotide hybridization' in der Anmeldung erw hnt
- NUCLEIC ACIDS RESEARCH Bd. 18, Nr. 22, 1990, ARLINGTON, VIRGINIA US Seite 6739 Y. JINNO ET AL. 'Use of psoralen as extinguisher of contaminated DNA in PCR'

## Beschreibung

Gegenstand der vorliegenden Erfindung ist ein Verfahren zur Bestimmung von mindestens einer in vitro amplifizierten Nukleinsäure in einem Reaktionsraum.

Die moderne Analytik greift zunehmend auf molekularbiologische Grundprinzipien zurück. Mit der Polymerase Ketten Reaktion (PCR) ist in jüngster Zeit ein sehr nützliches Werkzeug für die Analytik mittels molekularbiologischer Grundlagen gefunden worden, die sich auf allen Gebieten der Analytik, in denen Nukleinsäuren eine direkte oder indirekte Rolle spielen, angewendet werden kann.

Die PCR-Analytik (R.K. Saiki et al. (1988) Science 239, 487 - 491) und andere enzymatische Amplifikationstechniken (J.C. Guatelli et al. (1990) Proc. Natl. Acad. Sci. 87, 1874 - 1878) sind prinzipiell in der Lage, niedrige Titer von DNA- oder RNA-Kopien in einer wäßrigen Lösung nachzuweisen. Letztlich ist eine einzige Kopie ausreichend für eine enzymatische Amplifikation. Allerdings haben sich eine Reihe praktischer Schwierigkeiten bei der routinemäßigen qualitativen Anwendung herausgestellt sowie bei dem Bestreben, PCR mit einer quantitativen Kopienzahl-Bestimmung zu verbinden. Die Schwierigkeiten der Amplifikationsanalytik stehen im Zusammenhang mit:
- Unerwünschter Amplifikation durch Fehlpriming,
- ungleichmäßiger Verbrauch der Primer,
- Heteroduplex-Bildung,
- schwankende Zykluseffizienzen,
- diverse Amplifikationsartefakte,
- Quantifizierungsproblematik,
- Kontaminationsgefahr,
- "falsch positve" Resultate,
- "falsch negative" Resultate,
- Testkosten,
- Serienreife,
- Aufwand in der Handhabung, verglichen mit immunologischen ELISA-Verfahren für die Proteinanalytik.

Die WO 90/15881 beschreibt eine Methode zur Detektion spezifischer Nukleinsäuresequenzen in biologischen Proben unter Durchführung der folgenden Verfahrensschritte, enzymatische Amplifikation der Nukleinsäuren unter Verwendung geeigneter Primer, Verdünnung der angereicherten Amplifikationslösung, weitere Amplifikation und Detektion der erhaltenen Nukleinsäurekopien.

Hearst (Ann. Rev. Phys. Chem. 1988, 39: 291-315) beschreibt die photochemische Untersuchung der Dynamik von Oligonukleotidhybridisationen insbesondere unter Verwendung von Psoralenderivaten.

Die WO 90/01563 beschreibt eine Hybridisierungsmethode, mit der komplementäre und teilkomplementäre DNA-Basensequenzen unterschieden werden können, unter Verwendung von vernetzenden Reagentien wie Psoralenderivaten.

Die WO 91/02815 beschreibt einen sicheren qualitativen und quantitativen Nachweis spezifischer DNA und RNA aus biologischem Probenmaterial mit Hilfe einer DNA/RNA-Amplifikationsmethode in Kombination z. B. mit der Temperaturgradienten-Gelelektrophorese (siehe auch K. Henco & M. Heibey (1990) Nucleic Acids Res. 19, 6733 - 6734, J. Kang et al. (1991) Biotech Forum Europ 8, 590 - 593, G. Gilliland et al. Proc. Natl. Acad. Sci. (1990) 87, 2725 - 2729).

Mit der in der WO 91/02815 beschriebenen Amplifikationsstrategie ist es gelungen, die Sensitivität der Amplifikationstechniken mit einer sicheren und äußerst genauen Quantifizierung zu verbinden (Variation ± 15%). Die Methode ermöglicht es, die oben genannten Probleme, wie sie sich bei Amplifikationsverfahren ergeben, zu kontrollieren bzw. zu unterdrücken. Mit der Verwendung eines nahezu idealen internen Standards definierter Kopienzahl, der sich nur in einer einzigen Basenposition von dem zu bestimmenden Template unterscheidet, läßt sich eine Amplifikation unter Beibehaltung des initialen Verhältnisses von Template und Standard durchführen (K. Henco & M. Heibey ((1990) Nucleic Acids Res. 19, 6733 - 6734). Template und Standard werden nachträglich in einem Temperaturgradientenverfahren mit zeitlichem oder räumlichem Temperaturgradienten im gelelektrophoretischen System durchgeführt. Das Verhältnis von Template und Standard läßt sich einfach ermitteln, indem das Reaktionsgemisch mit einem radioaktiv markierten oder fluoreszenz-markierten Standard hybridisiert wird. Die ansonsten störend auftretende Heteroduplex-Bildung kann in diesem System das Ergebnis nicht verfälschen. Vielmehr wird die Heteroduplex-Bildung zur eigentlichen Messung herangezogen.

Übliche Probleme durch ungleichmäßigen Verbrauch der Primer, Fehlpriming-Reaktionen, Sättigungseffekte, Variationen in der Effektivität der Amplifikationen, haben keinen negativen Einfluß auf das Quantifizierungsergebnis.

Diese Technologie wurde auf wichtige analytische Fragestellungen in der Medizin angewandt. So ist es beispielsweise möglich geworden, die Cytomegalie-Infektion bzw. -Virämie bei transplantierten Patienten oder Neugeborenen quantifizierend zu erfassen. Hier hat sich das Verfahren besonders bewährt, denn angesichts einer stellenweise mehr als 90%-igen Infektionsrate in den europäischen Staaten ist der reine Nachweis von Cytomegalie-Viren nicht bedeutungsvoll, sofern nicht gleichzeitig eine Titer-Bestimmung durchgeführt wird, die den akuten Zustand einer Virämie anzeigt. Bei viralen Erkrankungen gewinnt der quantifizierende Aspekt an Bedeutung, insbesondere im Zusammenhang mit den im verstärkten Maße angewandten antiviralen Therapie-Schemata. Häufig sind antivirale Therapien, wie zum Beispiel im Falle von HIV mit AZT für den Patienten systemisch äußerst belastend und als antivirales Therapeutikum nur über einen limitierten Zeitraum erfolgreich einzusetzen. Deshalb wird es für die Zukunft sehr bedeutsam sein, eine wirtschaftliche und einfache Technik zu haben, die sowohl eine Titer-Bestimmung bzw. eine Bestimmung der Virusgen-Aktivität oder einen Therapie-induzierten Drift zu resistenten Virusstämmen anzeigt.

Die bislang angewandten gelelektrophoretischen Methoden zur Auftrennung der markierten Homoduplices und Heteroduplices haben sich bei kleinen bis niedrigen Probenzahlen (10 - 20) als äußerst effizient und korrekt bezüglich der gelieferten Ergebnisse herausgestellt. Ein gravierender Nachteil ist jedoch die relativ zeit- und personalaufwendige Analysentechnik, die den Einsatz dieses sehr aussagefähigen Analyseverfahrens als generelle Diagnostik-Methode für die DNA- und RNA-Analytik erschwert. Es ist mit der Temperatur-Gelelektrophorese-Technik kaum möglich, Probenzahlen > 50 pro ausführender Person und Tag durchzuführen. Auch eine Automatisierung ist nur schwer zu erreichen.

Aufgabe der vorliegenden Erfindung ist es mithin, ein Verfahren bereitzustellen, daß die oben erwähnten Nachteile der Verfahren gemäß dem Stand der Technik vermeidet, insbesondere ein Nachweisverfahren für Nukleinsäuren so auszugestalten, daß keine gelelektrophoretische Trennung erforderlich ist, um somit eine einfachere und automatisierbare Verfahrensweise zu gewährleisten.

Erfindungsgemäß gelöst wird die Aufgabe durch ein Verfahren zur qualitativen und quantitativen Bestimmung von mindestens einer in vitro-amplifizierten Nukleinsäure in einem verschlossenen Reaktionsraum,
- wobei während oder nach der Amplifizierung der Nukleinsäure mindestens eine Sonde vorhanden ist, die mit der nachzuweisenden Nukleinsäure in Wechselwirkung tritt,
- wobei spektroskopisch meßbare Parameter der Sonde eine Änderung erfahren und dabei ein meßbares Signal erzeugt wird,
- die zu messende Probe der Einwirkung eines Gradienten ausgesetzt wird, der Nukleinsäure mindestens teilweise denaturieren kann
- unter Erfassung des sich durch die Einwirkung des Gradienten ändernden meßbaren Parameters und
- die gesamte Amplifikationsreaktion einschließlich der qualitativen und quantitativen Bestimmung in einem verschlossenen Reaktionsraum ohne zwischenzeitliche Öffnung durchgeführt wird.

Die Substanz (Sonde), deren Parameter spektroskopisch meßbar sein soll, enthält vorzugsweise mindestens einen fluoreszierenden Rest, vorzugsweise mit interkalierenden Eigenschaften und einen Nukleinsäureanteil. Die Wechselwirkung mit den in vitro amplifizierten Nukleinsäuren in Abhängigkeit des Denaturierungszustandes geht einher mit einer Änderung des spektroskopischen Meßsignals. Dies kann beispielsweise durch Interkalation des Farbstoffes in die Nukleinsäuredoppelhelix oder durch Verdünnungs- oder Konzentrierungseffekte im Reaktionsraum erfolgen.

In einer weiteren bevorzugten Ausgestaltung des erfindungsgemäßen Verfahrens wird der durch den Gradienten ausgelöste Denaturierungsprozeß der Nukleinsäure über eine Änderung der Wellenlänge und/oder eine Fluoreszenzintensität-Shift und/oder eine Änderung der Lebenszeit eines angeregten Zustands oder über das Prinzip des sogenannten Energy Transfers (Förster Transfer) oder über einen Konzentrationseffekt erfaßt oder über unterschiedliche, vorzugsweise hydrophobe Wechselwirkungseigenschaften der markierten Sonde.

Das erfindungsgemäße Verfahren ermöglicht auch eine simultane oder sequenzielle Erfassung mehrerer verschiedener amplifizierter Nukleinsäuren. Dies erfolgt dadurch, daß mehrere spektroskopisch voneinander unterscheidbare Farbstoffe eingesetzt werden, mit denen sich die verschiedenen amplifizierten Nukleinsäuren analysieren lassen und/oder über die mindestens eine unabhängige Eichsubstanz eingeführt wird. Dies ist insbesondere dann möglich, wenn die verschiedenen Nukleinsäuren, die analysiert werden sollen mit unterschiedlich markierten Hybridisierungspartnern in Wechselwirkung treten.

Die Aufnahme des Meßsignals erfolgt beispielsweise durch Messung der von den Farbstoffen ausgehenden Fluoreszenz, welche insbesondere durch einen Laser kontinuierlich oder getaktet angeregt werden können.

Eine weitere bevorzugte Ausgestaltung des erfindungsgemäßen Verfahrens beruht darauf, daß die amplifizierten Nukleinsäuren mindestens einen koamplifizierten Nukleinsäurestandard enthalten, dessen Sequenz zu einer zu bestimmenden Sequenz homolog ist, vorzugsweise identisch ist, mit Ausnahme jedoch mindestens einer Punktmutation, die insbesondere in einem Sequenzbereich niedrigster Stabilität liegt. Es ist jedoch darauf zu achten, daß diese Punktmutation außerhalb der Primer-Bindungsstellen liegt, wenn enzymatische Amplifikationen durchgeführt werden. Der Nukleinsäurestandard kann ebenfalls ein natürlicher Bestandteil der zu analysierenden Nukleinsäure sein.

Erfindungsgemäß ist es auch möglich, eine erfolgreiche Amplifizierung einer bestimmten Nukleinsäure zu beobachten, ohne nach erfolgter Amplifikation ein markiertes Standardfragment dem Reaktionsansatz zuzufügen. Bei dieser bevorzugten erfindungsgemäßen Vorgehensweise werden insbesondere die zur Amplifikation erforderlichen Primer eingesetzt (EP-A-0 469 755). Diese hybridisieren dann an den entsprechenden Stellen in der Sequenz der betreffenden Nukleinsäuren. Die entsprechenden Sequenzen können zwischen den Primerstellen jedoch so unterschiedlich sein, daß beim Durchlaufen des Temperaturgradienten beide Sequenzen, die amplifizierte Test- und Standardsequenz, voneinander getrennt denaturieren und vorzugsweise dabei kooperativ denaturieren.

Dadurch wird eine Verwendung solcher Sequenzen ermöglicht, die eine derart große Sequenzabweichung aufweisen, daß eine Heteroduplexbildung nicht mehr erfolgen kann. Damit entfällt auch die Notwendigkeit, nach der erfolgten Amplifikation ein markiertes Standardfragment zusetzen zu müssen. Ein unterschiedlicher Schmelzpunkt beider Sequenzen läßt sich beispielsweise durch starke Längenvariation der Sequenz oder durch die Wahl einer Poly-A/T-Sequenz beeinflussen. Die Frage, ob eine Amplifikationsreaktion stattgefunden hat, läßt sich dann durch Anwendung des erfindungsgemäßen Verfahrens, zum Beispiel in einem Temperaturgradienten bei gleichzeitiger Anwesenheit von Ethidiumbromid entscheiden, wobei auch eine quantitative Erfassung ermöglicht wird.

Das erfindungsgemäße Verfahren erlaubt die Durchführung der Amplifikation in homogener Phase oder an fester Phase, vorzugsweise mit einem an eine feste Phase gekoppelten Primer, an dessen verlängerter Sequenz die markierte Sonde hybridisieren kann. Die Konzentration der Sonde kann somit entweder gezielt auf dem Festphasenträger oder in der freien Lösung bestimmt werden.

Vorzugsweise wird mindestens ein Fluoreszenzfarbstoffmolekül an ein Nukleinsäuremolekül gekoppelt, dessen Sequenz identisch oder homolog mit der nachzuweisenden Nukleinsäure oder dem koamplifizierten Nukleinsäure-standard ist.

Wenn das Nukleinsäuremolekül mit dem daran gekoppelten Fluoreszenzfarbstoff dem Reaktionsgemisch nach erfolgter Amplifikation zugesetzt wird, erfolgt die Hybridisierung mit den amplifizierten Nukleinsäuren, vorzugsweise durch einen thermischen Denaturierungsschritt mit nachfolgendem Renaturierungsschritt. Es ist erfindungsgemäß jedoch auch möglich, daß das Nukleinsäuremolekül mit gekoppeltem Fluoreszenzfarbstoff dem Reaktionsgemisch vor erfolgter Amplifikation zugesetzt wird. Dabei ist die Sonde als nicht amplifizierbare Doppelstrang-RNA oder als nicht amplifizierbare chemisch modifizierte Nukleinsäure zuzusetzen.

Zur Amplifikation der Nukleinsäure wird in einer möglichen Ausführungsform ein Primer des zur Amplifikation eingesetzten Primerpaares verwendet, welcher 5'-terminal eine G:C-reiche Region enthält von beispielsweise 15 bis 20 G:C-Resten.

Die bislang beschriebene Standardisierung und Quantifizierung des erfindungsgemäßen Verfahrens durch die vorstehend bevorzugten Ausführungsformen der beispielsweise fluoreszierenden Sonden ist mit einer tragbaren Einschränkung behaftet. So dürfen die zur Standardisierung verwendeten Sonden erst nach erfolgter Amplifikation zugesetzt werden. Dies bedeutet, daß sie zunächst während der Amplifikationsreaktion räumlich vom Amplifikationsgeschehen getrennt aufzubewahren sind. Ebenso kann es für verschiedene Anwendungen vorteilhaft sein, Sonden zur Verfügung zu stellen, die als Standardnukleinsäuren verwendet werden und in mehr als einer Position von der zu bestimmenden Nukleinsäure abweichen. Um das Signal-Rausch-Verhältnis bei der späteren Bestimmung mit Hilfe der eingesetzten Sonde zu verbessern, ist es wünschenswert, die Sonde nicht im Unterschuß dem zu amplifizierenden Gemisch zusetzen zu müssen.

In einer besonders bevorzugten Ausführungsform des erfindungsgemäßen Verfahrens wird daher als Sonde ein Oligo- oder Polynukleotid als Einzelstrangnukleinsäure zugesetzt, welche durch chemische Modifizierung aber nicht an der Amplifizierungsreaktion teilnehmen kann. Erst durch geeignete Manipulationen wird nach der Amplifizierungsreaktion die einzelsträngige Sonde freigelegt und kann dann mit den entsprechenden zu bestimmenden Nukleinsäuren hybridisieren. So kann beispielsweise die Sonde, wenn sie in Form einzelsträngiger Nukleinsäure vorliegt, in Form einer "Haarnadelstruktur" inaktiviert werden und somit an der Teilnahme an der Amplifikationsreaktion gehindert werden.

Die als Sonden in besonders bevorzugter Weise zu verwendenden Oligo- oder Polynukleotide weisen ein oder mehrere Strukturelemente mit mindestens zwei chemischen Substituenten auf, die jeweils in der Lage sind, mit elektromagnetischen Wellen unter Lösen oder Knüpfen dauerhafter Bindungen oder durch Absorption und/ oder Emission von Strahlung in Wechselwirkung zu treten. Als Substituent, der besonders geeignet ist, unter Knüpfen und Lösen von dauerhaften Bindungen, insbesondere kovalenten Bindungen, mit elektromagnetischer Strahlung in Wechselwirkung zu treten, hat sich Psoralen oder dessen Derivate bewährt. Als Strukturelemente, die als eigentliche Marker der Sonde dienen, haben sich insbesondere Lumineszenzfarbstoffe, wie Fluoreszenzfarbstoffe mit hoher Quantenausbeute bewährt, wie die Farbstoffe der Thiazolorange-Familie. Farbstoffe mit großen Stoke-Verschiebungen sind bevorzugt, die in Abhängigkeit vom Hybridisierungszustand die Lumineszenzeigenschaften ändern.

Es kann vorteilhaft sein, daß die Spektren der Strukturelemente an den jeweiligen sensitiven Stellen, die einerseits zum Lösen und Knüpfen von beispielsweise kovalenten Bindungen anzuregen sind, oder andererseits als Absorptions- oder Emissionsmaximum anzusehen sind, so weit auseinanderliegen, daß eine jeweilige Anregung die Funktion des anderen Strukturelementes nicht stört.

Es ist ebenfalls möglich, die beiden chemischen Strukturelemente in einer einzigen chemischen Struktur zu vereinen, sofern das Knüpfen und Lösen von Bindungen jeweils bei anderen Wellenlängen erfolgt, wie eine mögliche maximale Fluoreszenz dieser Struktur. So können die jeweiligen Funktionen, zum einen die Fixierung der Sonde in einer nicht amplifizierten Struktur und zum anderen die spektroskopische Identifizierung dieser Struktur, nicht miteinander interferieren.

Sofern zwei getrennte Strukturelemente die getrennten Funktionen wahrnehmen hat sich als vorteilhaft erwiesen, daß deren Abstand von mindestens 10 Nukleotiden auf dem Oligo- oder Polynukleotidstrang nicht unterschreiten sollten.

Die bevorzugte Ausführungsform des erfindungsgemäßen Verfahrens unter Verwendung der beschriebenen Oligo- und/oder Polynukleotide funktioniert vorzugsweise so, daß die Sonden dem bereits oben beschriebenen Gemisch von Substanzen zugefügt werden, die Amplifikationsreaktion wie beschrieben durchgeführt wird und danach beispielsweise durch Strahlung induziert, die verkappte Sonde freigesetzt wird, um dann mit dem zu bestimmenden Amplifikationsprodukt zu hybridisieren und wie beschrieben beispielsweise durch einen zeitlichen Temperaturgradienten in homogener Lösung, durch Temperaturgelelektrophorese oder einen chromatographischen Verfahren detektiert zu werden.

Mit dieser Variante des erfindungsgemäßen Verfahrens wird es möglich, die Analyse so durchzuführen, daß die markierte Hybridisierungssonde nicht mehr innerhalb des Kompartimentes des Reaktionsraumes separat angeordnet sein muß, um sie dem Reaktionsgemisch erst unmittelbar vor der eigentlichen Analyse zuzusetzen. Es ist somit möglich, die markierte Sonde mit den übrigen Reagenzien in einer Form vorzulegen, in der sie selbst nicht am nachfolgenden Amplifikationsprozeß teilnehmen kann. Dadurch wird eine Trennung des Amplifikationsgemisches und der Sonde innerhalb des Meßraumes vermeidbar.

Zur Durchführung des Verfahrens werden vorzugsweise folienartige Systeme mit Vertiefungen oder Ausbuchtungen, die als Reaktionsräume (Kompartimente) dienen, verwendet. Die Foliensysteme sind vorzugsweise thermisch verschweißbar und zur Aufnahme verwendungsfertiger Reagenziengemische in gefriergetrockneter oder matrixgebundener Form geeignet. Weiterhin ist eine direkte optische Vermessung des Inhalts der Reaktionsräume möglich. Das Folienmaterial ist also zumindest für bestimmte Wellenlängenbereiche elektromagnetischer Strahlung durchlässig bzw. transparent. Die zur Durchführung des erfindungsgemäßen Verfahrens benötigten Reagenzien werden vorzugsweise in räumlich abgetrennten Matrices gelagert und nach dem Schließen eines Reaktionsraumes zeitlich getrennt dem Reaktionsgeschehen zugeführt. Vorzugsweise sind die Reaktionsräume im Abstand der Löcher handelsüblicher Mikrotiterplatten voneinander getrennt. Dies hat insbesondere den Vorteil, daß Geräte, die zur Bearbeitung von Mikrotitrationsplatten geeignet sind, in der erfindungsgemäß beschriebenen Technologie eingesetzt werden können.

Zur Analyse eines amplifizierten Nukleinsäuregemisches wird nach Zugabe der zur Reaktion benötigten Substanzen vorzugsweise ein zeitlich gesteuerter Temperaturgradient angelegt und das Denaturierungsverhalten der Nukleinsäuren gemessen. Dies erfolgt durch die Änderung der spektroskopischen Parameter der mit der Nukleinsäure in Wechselwirkung tretenden Substanz. Die Änderung des spektroskopischen Parameters wird zeitlich oder in äquivalenter Weise, in Abhängigkeit der Temperaturänderung verfolgt.

Die Auswertung der Funktion der Änderung des spektroskopischen Verhaltens der mit der Nukleinsäure in Wechselwirkung tretenden Substanz erlaubt dann den Schluß auf Anwesenheit oder Anzahl oder Homologie einer gesuchten Nukleinsäure zum korrespondierenden Standard. Die Auswertung der Daten erfolgt vorzugsweise on line durch eine Datenverarbeitungsanlage.

Vorteilhaft am erfindungsgemäßen Verfahren ist neben dem Abstellen der oben genannten Nachteile des Standes der Technik, daß die Amplifizierung der Nukleinsäuren und die spätere Analytik in einem einzigen hermetisch verschlossenen Reaktionskompartiment durchgeführt werden kann. Dadurch wird eine Entsorgung dieser biologischen Stoffe ohne Öffnung der Kompartimente möglich und eine potentielle Kontaminationsquelle ausgeschaltet. Desweiteren ermöglicht diese Vorgehensweise die Lagerung von Testfolien der oben genannten Art im geschlossenen Zustand auch über längere Zeiträume, so daß eine Archivierung der oftmals wertvollen Testsubstanzen möglich wird. Die Lagerung erfolgt aber vorzugsweise im gefrorenen Zustand. Das erfindungsgemäße Verfahren ermöglicht ebenfalls in vorteilhafter Weise, daß die Versuche gegebenenfalls zu einem späteren Zeitpunkt auch nach längerer Zwischenlagerung wiederholbar sind, oder das amplifizierte Gemisch danach präparativ aufarbeitbar und analysierbar ist.

Die im erfindungsgemäßen Verfahren einsetzbare Vorrichtung weist eine Einrichtung zur zeitabhängigen Thermostatisierung der im Verfahren zu verwendenden Reaktionsräume auf. Vorzugsweise wird die Thermostatisierung durch eine programmierbare Einheit gesteuert. Die Ableseeinrichtung der erfindungsgemäßen Vorrichtung besteht vorzugsweise aus einer optischen Einheit, die in der Lage ist, Photonen zu registrieren. Besonders bevorzugt sind solche Einheiten, die zur Registrierung emittierten Fluoreszenzlichts geeignet sind. Ebenfalls in Betracht kommen Geräte, die in der Lage sind, andere spektroskopische Eigenschaften, wie Kernspin oder Elektronenspin etc., die mit der Änderung der Konformation der Nukleinsäure-Doppelhelix oder anderen Strukturvariablen korrelierbar sind, oder chromatographische Verfahren einzusetzen. Mit der Methode der Chromatographie hydrophober Wechselwirkungen lassen sich Moleküle mit hydrophoben Liganden, wie sie partiell denaturierende Strukturen der zu analysierenden Substanzen darstellen, von den Dublices abtrennen.

Die im erfindungsgemäßen Verfahren einsetzbare Vorrichtung ist in der Lage, ein System von Reaktionskompartimenten, vorzugsweise aus einem Foliensystem mit gebrauchsfertigen Reagenzien in gefriergetrockneter Form aufzunehmen. Die Reaktionskompartimente sind vorzugsweise im Mikrotitrationsformat angeordnet. Die Reagenzien sind vorzugsweise in mindestens einer wasserlöslichen Matrix fixiert und/oder gelagert. Die Matrix enthält vorzugsweise Stabilisatoren, wie Zucker, insbesondere Trehalose oder Saccharose. Bevorzugt werden Reaktionskompartimente und/oder weitere Reagenzienreservoire, Amplifikationsprimer, Pufferbestandteile sowie mindestens eine Polymerase und übliche Kofaktoren zur Durchführung der Amplifikationsreaktion im erfindungsgemäßen Verfahren eingesetzt. vorzugsweise ist der Reaktionsraum bzw. das Reaktionskompartiment mit einem weiteren abgetrennten Reagenzienreservoir in einer Matrix versehen, die sich in der das Kompartiment verschließenden Folie befindet. Dabei wird vorzugsweise die markierte Sonde mit den für die Hybridisierung notwendigen Puffersubstanzen gelagert.

Reaktionsgefäße, wie Foliensysteme mit lagerfähigen und direkt einsetzbaren Reagenziengemischen, wobei die Reaktionsgefäße nur mit der zu analysierenden Probe beschickt werden müssen, um dann im hermetisch versiegelten Zustand einem Amplifikationsverfahren und anschließender Analyse unterworfen zu werden, werden insbesondere in Form von Kit-Systemen eingesetzt. Das erfindungsgemäße Verfahren eignet sich insbesondere zur Analyse von Stoffgemischen, vorzugsweise Nukleinsäuren, in denen mindestens eine Komponente im Temperaturbereich des zeitlichen Temperaturgradienten eine thermische Umwandlung erfährt. Durch Hinzufügen und Koamplifikation eines Standards mit genau bekannter Kopienzahl ist das erfindungsgemäße Verfahren standardisierbar und erlaubt quantitative Aussagen über die amplifizierten Nukleinsäuren der zu untersuchenden Probe. Mit dem erfindungsgemäßen Verfahren lassen sich zum Beispiel Mutationen, Punktmutationen, Deletionen, Insertionen und Umstellungen in der DNA/RNA-Nukleinsäure feststellen. Mit der quantitativen Analyse läßt sich auch die Konzentration dieser Änderungen in der Nukleinsäure ermitteln. Die Proben können aus unterschiedlichstem Material stammen, wie lebendem, totem, fossilem und in vivo nicht mehr stoffwechselaktivem Gewebe oder aus Körperflüssigkeiten, aus in vitro Zellkulturen oder aus Proben der Umwelt. Das erfindungsgemäße Verfahren ermöglicht die qualitative und quantitative Erfassung zellulärer Gene und Gene infektiöser Krankheitserreger direkt oder über ihre RNA-Genprodukte als Wildtypsequenz oder als Varianten.

Das erfindungsgemäße Verfahren läßt sich aber auch zur Untersuchung und Bestimmung von potentiell toxischen Substanzen oder potentiell pharmazeutischen Wirkstoffen oder chemischen oder biologischen Pflanzenschutzmitteln einsetzen, indem deren Einfluß auf Nukleinsäuren oder deren Amplifikation in zellulären oder nicht zellulären Systemen untersucht werden.

### Figur 1

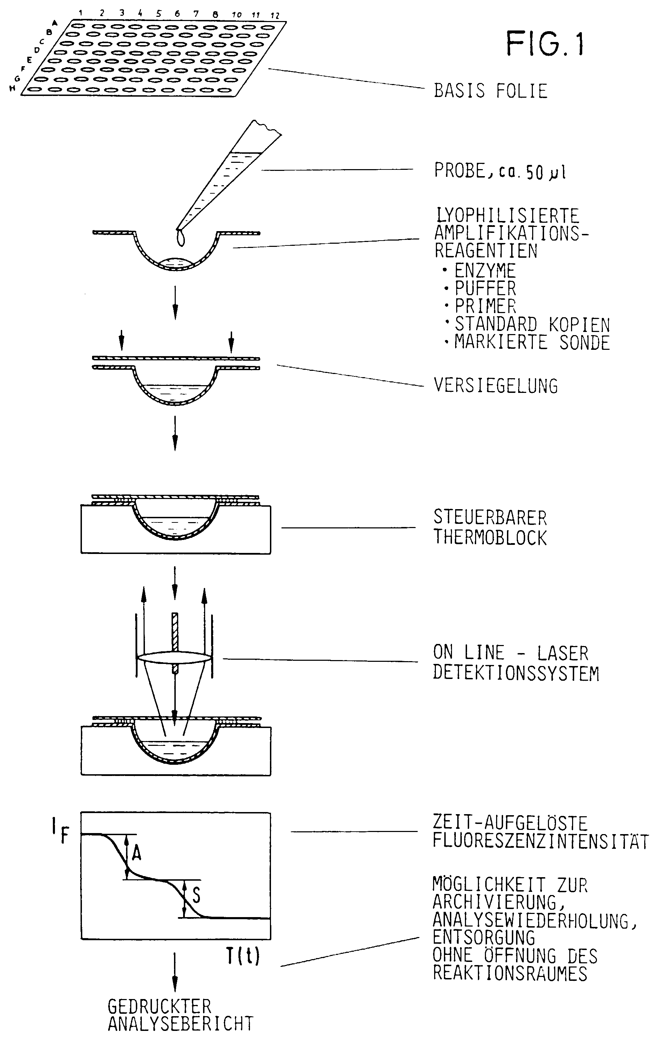

Die Figur 1 beschreibt schematisch einen bevorzugten Ablauf des erfindungsgemäßen Verfahrens. In einer Vorlage von Reaktionskompartimenten in Format einer Platte für die Mikrotitration befinden sich die für eine spezifische Amplifikation einer Nukleinsäure notwendigen Reagenzien, zum Beispiel in lyophilisierter Form inklusive der Sonde. Lediglich die zu analysierende Probe wird beispielsweise in wäßrig-gelöster Form vor der Amplifikationsreaktion zugesetzt. Es wird eine homogene Lösung hergestellt.

Danach werden die Reaktionskompartimente mit einer zweiten Folie verschlossen, wobei die zweite Folie mindestens eine weitere Matrix enthält mit Reagenzien, die nicht an der eigentlichen Amplifikationsreaktion teilnehmen sollen. Die Folie wird in einem Thermostatierblock positioniert, um die enzymatische Amplifikationsreaktion durchzuführen. Hierbei können sowohl Amplifikationen bei homogener Temperatur als auch in zyklisch variierenden Temperaturabläufen (PCR) ausgeführt werden. Nach erfolgter Amplifikation wird das Reaktionsgemisch mit dem zweiten Reagenzienreservoir in Kontakt gebracht und eine homogene Lösung hergestellt. Nach Durchführen eines Denaturierungs-/Renaturierungsprozesses registriert ein optisches Detektionssystem die laserangeregte Lumineszenz (Fluoreszenz, Phosphoreszenz) in Abhängigkeit eines linearen Temperaturgradienten, der über den Thermoblock zeitlich gesteuert wird. Der steigende Temperaturgradient kann in einem weiten Bereich variiert werden. Die Anfangstemperatur kann z. B. minimal 5°C, die Endtemperatur maximal 100 °C betragen.

### Figur 2

Die Darstellung zeigt in schematischer Form den Verlauf des erfindungsgemäßen Verfahrens am Beispiel interkalierender Farbstoffe auf molekularer Ebene, die nicht an eine Nukleinsäuresonde fixiert sind (z.B. Ethidiumbromid oder Thiazolorange-Farbstoffe). Diese Farbstoffmoleküle lagern sich unter nativen Bedingungen zwischen benachbarte Basenpaare in doppelsträngiger DNA oder RNA ein. Im interkalierten Zustand erhöht sich die Fluoreszenzausbeute bis zum 20-fachen und die Lebensdauer des angeregten Zustandes etwa um das 10-fache. Wird dieses System einem thermischen Gradienten unterworfen, so beginnen zunächst die thermodynamisch instabilsten Bereiche der Nukleinsäurehelix zu denaturieren. Eine Fehlpaarung, wie sie bei der Heteroduplexbildung erzeugt wird, destabilisiert den entsprechenden Sequenzbereich und führt zu dessen frühzeitiger Öffnung. Die in diesem Bereich zunächst gebundenen Farbstoffmoleküle werden freigesetzt, wodurch sich eine Erniedrigung des Gesamtfluoreszenzsignals ergibt. Dabei ist die Farbstoffkonzentration so zu wählen, daß freier Farbstoff und gebundener Farbstoff im thermodynamischen Gleichgewicht stehen und der freie Farbstoff in deutlichem Überschuß vorliegt. Erst bei höheren Temperaturen öffnet sich der entsprechende Sequenzbereich des Homoduplex, wodurch eine weitere stufenweise Erniedrigung des Fluoreszenzsignals erfolgt. Aus dem Verhältnis der Intensitäten beider Stufen lassen sich die relativen Mengenverhältnisse von Homoduplex und Heteroduplex ermitteln.

### Figur 3a und 3b

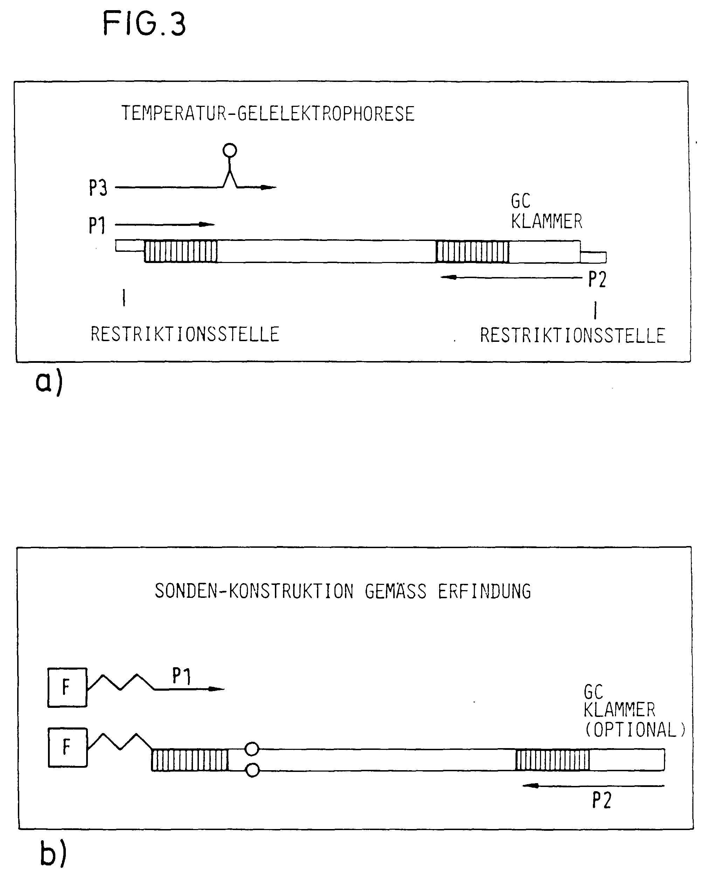

Die Figur 3a zeigt das Design eines Standards bzw. einer fluoreszenzmarkierten Sonde in einer bevorzugten Ausführungsform. Der Standard soll sich in mindestens einer Basenposition von der nachzuweisenden Nukleinsäure unterscheiden. Diese Mutation befindet sich vorzugsweise in dem Sequenzbereich niedrigster thermodynamischer Stabilität und kann leicht unter Verwendung des Primers P3 eingeführt werden. Die Mutation soll sich jedoch außerhalb der Primer-Bindungsstellen von P1 und P2 befinden. Ein Primer (P2) kann 5'-terminal eine GC-reiche Region enthalten, während der andere Primer (P1) vorzugsweise mit einem Fluoreszenzfarbstoff über einen Spacer chemisch gekoppelt ist, wobei der Fluoreszenzfarbstoff im Bereich der Doppelhelix interkalieren kann. Das Anwendungsprinzip der fluorezenzmarkierten Sonde ist in Figur 3b schematisiert dargestellt.

### Figur 4

in einer bevorzugten Ausführungsform des erfindungsgemäßen Verfahrens werden Primer eingesetzt, wie beschrieben in: Thuong, N.T. & Chassignol, M. (1987) Tetrahedron Letters 28, 4157-4160; Thuong, N.T. et al. (1987) Proc. Natl. Acad. Sci. U.S.A. 84, 5129-5133; Helene, C. in DNA-Ligand Interactions (1987) Plenum Publishing Corporation, 127-140, W. Gushelbauer & W. Saenger Ed. Sie sind bereits teilweise kommerziell erhältlich (Appligene, Straßburg, Frankreich) Er enthält einen Spacer-gekoppelten Fluoreszenzfarbstoff, der interkalationsfähig ist, wenn sich der Primer in einem doppelhelikalen Bereich befindet. Wird die Doppelhelix thermisch denaturiert, so ändern sich die Fluoreszenzeigenschaften des Farbstoffes.

### Figur 5

Die Figur zeigt schematisch eine bevorzugte Herstellung der im erfindungsgemäßen Verfahren verwendbaren Sonden, die modifiziert sind und nicht an der Amplifikationsreaktion teilnehmen.

Die europäischen Patentanmeldungen EP 469 755 und EP 379 369 beschreiben die Erzeugung von Nukleinsäurestrukturen. Dort wird beispielhaft eine Struktur erzeugt, deren Enden zueinander komplementär sind. Hierfür kommen zunächst die Primer pl und (pl) zum Einsatz. Zur Herstellung der Sonden wird aus dem entsprechenden Amplifikat des Standards mit einem Primer pl, der das Strukturelement, welches dauerhafte Bindungen zu knüpfen vermag ("chemische Klammer"), ein Amplifikat hergestellt.

pl wird vorzugsweise so gewählt, daß auf der 3'-Seite eine Restriktionsschnittstelle mit einem 5'-überhängenden Ende entsteht. Somit entsteht nach Restriktionsschnitt eine Struktur, die mit Nukleotiden aufgefüllt werden kann, welche ihrerseits beispielsweise einen Chromophor tragen, der der eigentliche Marker der Sonde ist. Da nach diesem Herstellungsverfahren beide Stränge den Farbstofflabel und die "chemische Klammer" enthalten, wird eine Strangtrennung und Isolierung erforderlich. Dies verursacht jedoch keine Probleme und kann beispielsweise mit einer präparativen Hochdruckflüssigkeitschromatographie erfolgen.

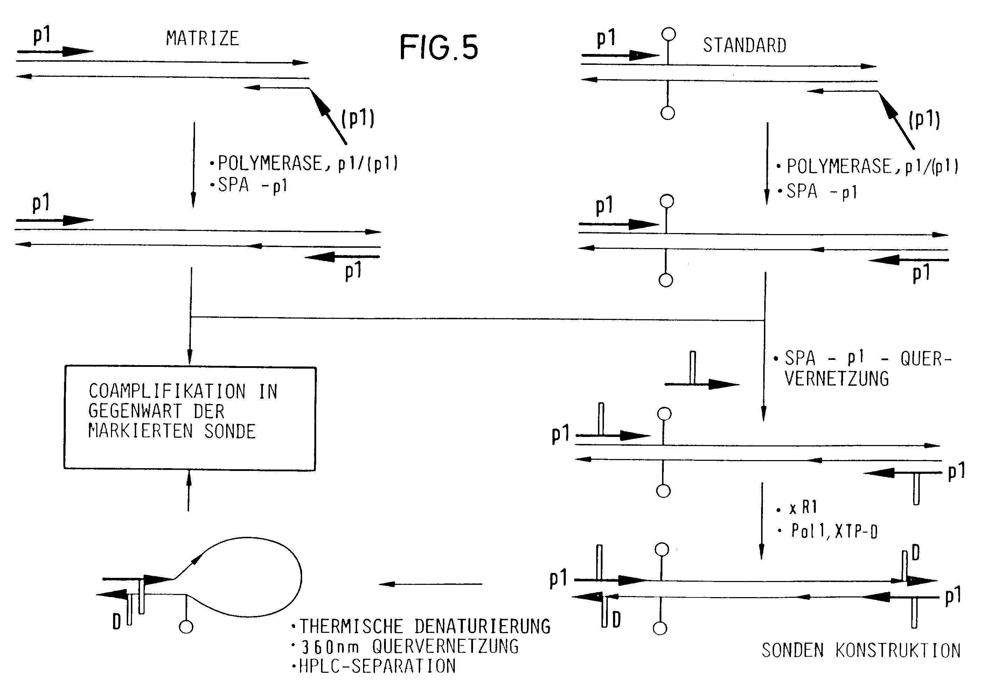

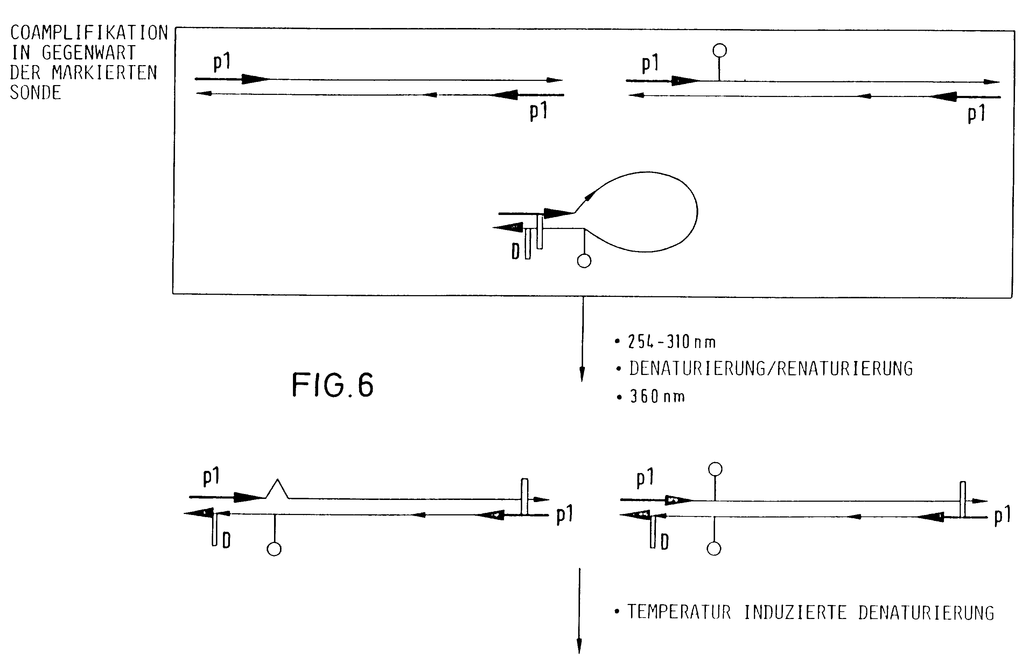

Die chemische Verknüpfung der komplementärhybridisierenden Enden der Einzelstrang-Haarnadelstruktur wird danach photochemisch oder chemisch induziert und verhindert somit die Template-Aktivität dieser Sonde während der enzymatischen Amplifikationsphase, wie sie in Figur 5 ebenfalls dargestellt ist. Die in Form eines Haarnadel-Loops vorliegende Sonde kann aufgrund ihrer verkappten Struktur in die Amplikation nicht mit einbezogen werden. Nach erfolgter Amplifikation kann beispielsweise bei Verwendung von Psoralenderivaten als "chemische Klammer" im kurzwelligen UV-Bereich die Verklammerung wieder gelöst werden. So wird der Sondeneinzelstrang im Denaturierungs-/Renaturierungsprozeß wieder freigesetzt und kann durch wiederholte Bestrahlung bei 360 nm, sofern Psoralenderivate verwendet werden, wieder verknüpft werden. Dieser Zusammenhang ist in Figur 6 schematisch dargestellt.

Wenn die chemische Verknüpfung mit dem Gegenstrang wiederum durch Strahlung induziert werden kann, kommt dieser chemischen Verknüpfung eine weitere vorteilhafte Funktion zu. Sie kann die Aufgabe der zuvor eingesetzten G/C-Klammern übernehmen und somit dafür sorgen, daß hybridisierte Stränge sich während der Analyse nicht mehr trennen können und somit eine irreversible Denaturierung ermöglicht wird. Wie bereits erwähnt, werden vorzugsweise als "chemische Klammern" Verbindungen mit Psoralengrundstruktur eingesetzt, welche die Eigenschaft besitzen, nach Aktivierung mit Licht mit einer Wellenlänge von 360 nm mit Pyrimidinbasen kovalent zu reagieren. Die Voraussetzung ist allerdings, daß die Pyrimidinbasen um eine Basenposition versetzt auf dem gegenüberliegenden Strang positioniert sind (J. E. Hearst (1988). An. Rev. Phys. Chem., 39, 291 bis 315).

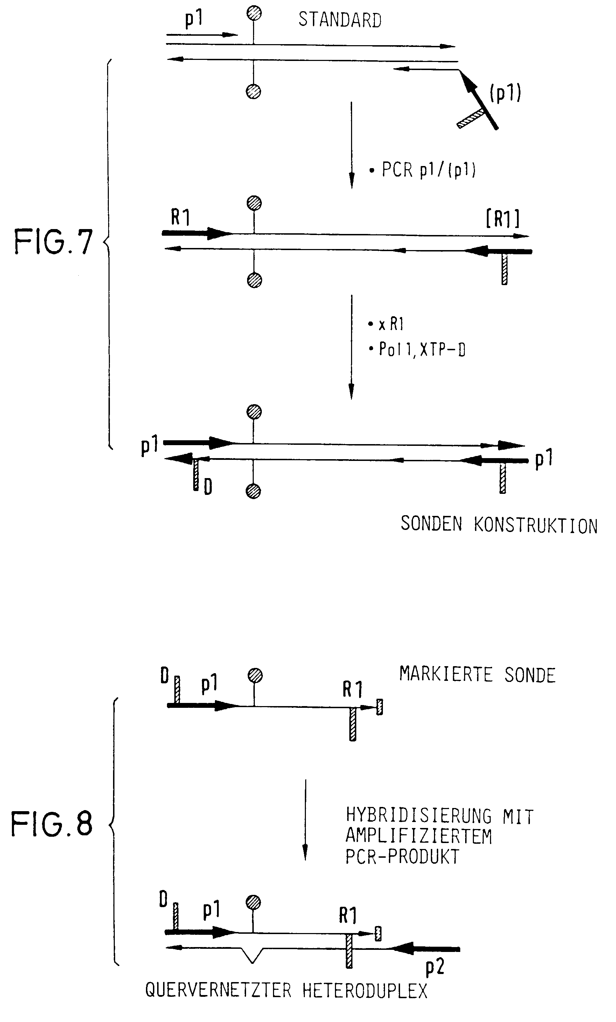

Eine andere Alternative zur Synthese der erfindungsgemäß zu bevorzugenden Sonde ist in Figur 7 gezeigt. Dabei wird die "chemische Klammer" über den Primer (pl) eingeführt. Somit trägt später lediglich der gewünschte Strang den chemischen Vernetzer. Durch den Vernetzer, der insbesondere photochemisch aktivierbar ist, wird in vorteilhafter Weise die benachbarte Schnittstelle des Restriktionsenzyms R1 inaktiviert. Auf diese Weise ist dafür Sorge getragen, daß anschließend der Farbstoff, der die eigentliche Markierung der Sonde besorgt, nur auf dem gewünschten Strang eingebaut wird. Ungünstig im Vergleich zur oben beschriebenen Sondenkonstruktion ist hier der etwas längere Primer (pl) mit Modifikation.

Eine dritte mögliche, bevorzugte Ausführungsform der erfindungsgemäß zu verwendenden Nukleinsäuresonde ist erhältlich durch den Einsatz einer einzelsträngigen Sonde, die ebenfalls einen quervernetzenden Liganden besitzt, dessen Position sich allerdings am 3'-Ende der Sonde befindet, während das Farbstoffmarkierungsmolekül am 5'-Ende eingebaut ist. Diese Situation wird in Figur 8 veranschaulicht. Die Sonde wird in diesem Falle so konstruiert, daß am 3'-Ende die Primerbindestelle fehlt, das 5'-Ende identisch mit der pl-Primersequenz ist, das 3'-Ende enzymatisch nicht verlängert werden kann und zwar dadurch, daß beispielsweise endständig ein Didesoxynukleotid eingebaut wird. Das quervernetzende Reagenz trennt räumlich die homologe Doppelstrangregion mit dem Markierungsstrukturelement von der diese nicht tragende Einzelstrangregion der Hybride. Wenn die Einzelstrangregion als Folge eines fehlerhaften Amplifikationsprozesses nicht einheitlich synthetisiert wird, ist dies für das Ergebnis der Sondenkonstruktion nicht beeinträchtigend.

Bei Verfolgung dieser Strategie der Sondenkonstruktion nimmt die Sonde deshalb nicht am Amplifikationsgeschehen teil, weil sie am 3'-Ende nicht verlängerbar ist, und somit selbst bei intermediär erfolgter Hybridisierung mit amplifiziertem Gegenstrang keine elongierte Primer-Bindungsstelle enzymatisch synthetisiert wird.

Nach erfolgter Amplifikation muß der vernetzende Ligand somit nicht zunächst aus einer vernetzten Struktur durch Bestrahlung herausgelöst werden um nachfolgend in der rehybridisierten Struktur mit Amplifikat wiederum vernetzt zu werden; es reicht ein einmaliger Bestrahlungsvorgang, um die Vernetzung mit dem Amplifikat zu bewirken.

### Figur 9

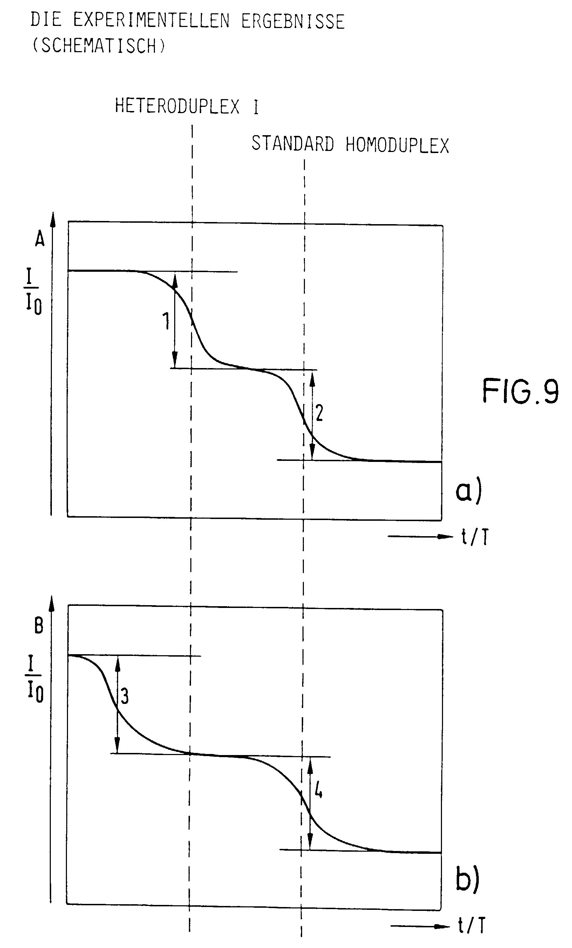

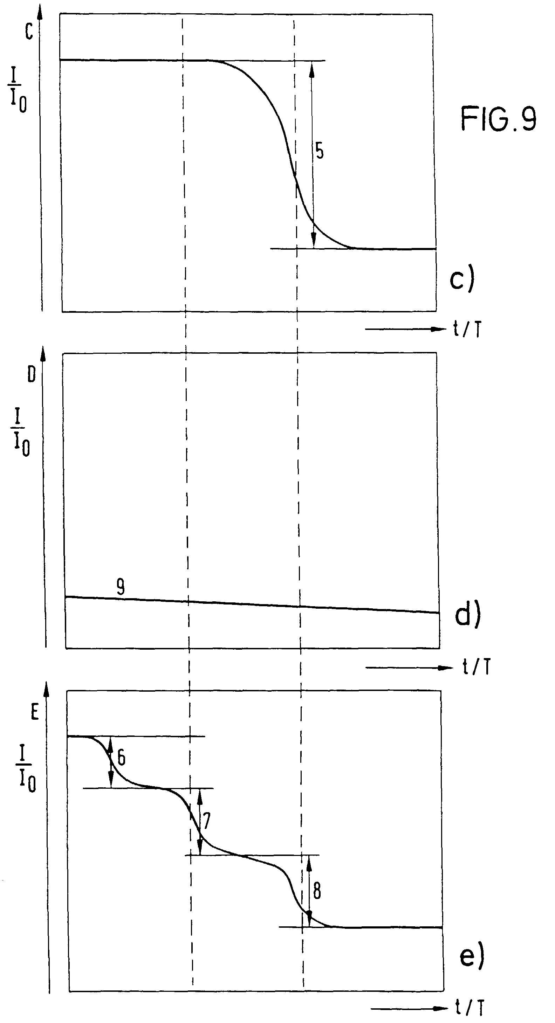

Die Figuren 9a - 9e zeigen schematisch mögliche experimentelle Resultate, die mit dem erfindungsgemäßen Verfahren erhalten werden können. Auf der Ordinate ist das Fluoreszenzsignal 1 bezogen auf die jeweilige Intensität (10) bei der niedrigsten experimentellen Temperatur (nativ) aufgetragen. Die X-Achse beschreibt den Temperaturverlauf des Aufheizungsvorgangs, der vorzugsweise zeitlich linear verläuft. Die zwei gestrichelten Linien markieren die Position des Standard Homoduplex (2) sowie die Position des aus markierter Sonde und zu erwartender WildtypSequenz (1) bestehenden Heteroduplex 1. Trägt die zu analysierende Sequenz mindestens eine weitere Mutation, so ergibt sich ein stufenweises Absinken des Fluoreszenzsignals bereits bei tieferer Temperatur, die für die Mutation charakteristisch ist (B3). Die relative Höhe der Stufen zueinander (1/2, 3/4, 6/7/8) gibt direkt das Verhältnis der relativen Konzentration der beteiligten Moleküle wieder. Ein Ergebnis gemäß Abbildung C wird erhalten, wenn lediglich die Standard-Nukleinsäure amplifiziert wird und die biologische Probe keine oder nur geringe Mengen der entsprechenden Nukleinsäure enthält. Ein Ergebnis gemäß D6 wird erhalten, wenn die Amplifikationsreaktion nicht ordnungsgemäß abgelaufen ist und nicht einmal der Standard amplifiziert worden ist. Enthält die biologische Probe mehr als eine homologe Nukleinsäurespezies so ergeben sich mehrere voneinander unterschiedliche Stufen des temperaturabhängigen Fluoreszenzverlaufes (6 und 7).

### Figur 10

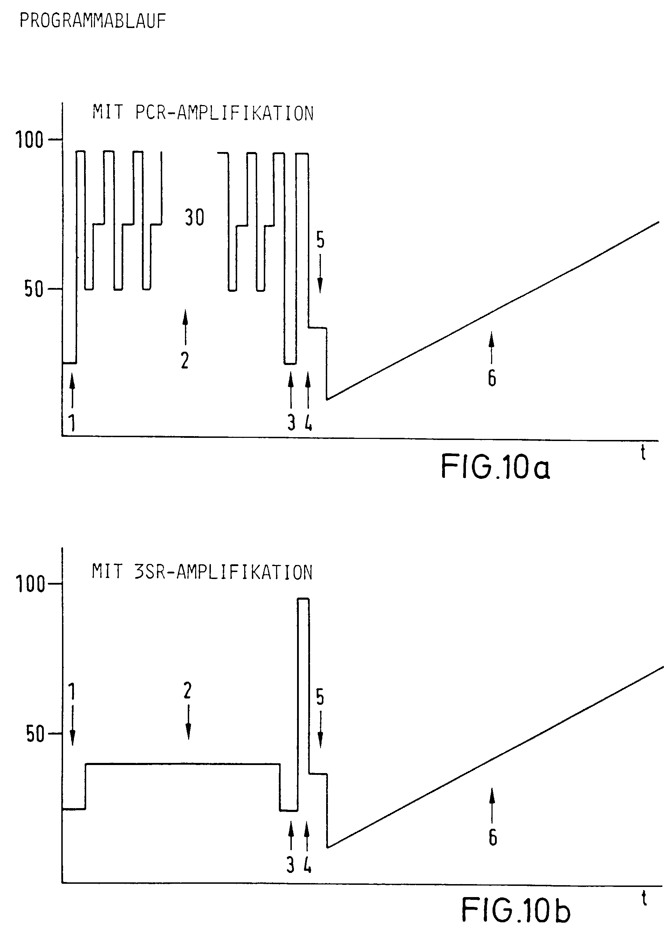

Die Figur 10 beschreibt den schematischen Temperaturverlauf und die zugehörigen Einzelschritte bei dem erfindungsgemäßen Verfahren. Im oberen Teil der Figur ist der Ablauf der Analyse mit der PCR Technik dargestellt, im unteren Teil der Figur der Verlauf bei einer Amplifikation bei homogener Temperatur (37°C), z. B. bei der 3SR-Technik (siehe unten).
1) Zusatz der biologischen Probe zum lyophilisierten Amplifikationsansatz und Verschweißen des Reaktionskompartimentes.
2) Amplifikation bei homogener Temperatur oder mit Temperaturprogrammen (PCR).
3) Zumischen der markierten Sonde(n) im Hybridisierungspuffer.
4) Denaturierungsschritt bei 98°C.
5) Sondenreassoziation mit der amplifizierten DNA.
6) Zeitlicher Temperaturgradient mit optischer On-Line-Kontrolle.

### Figur 11

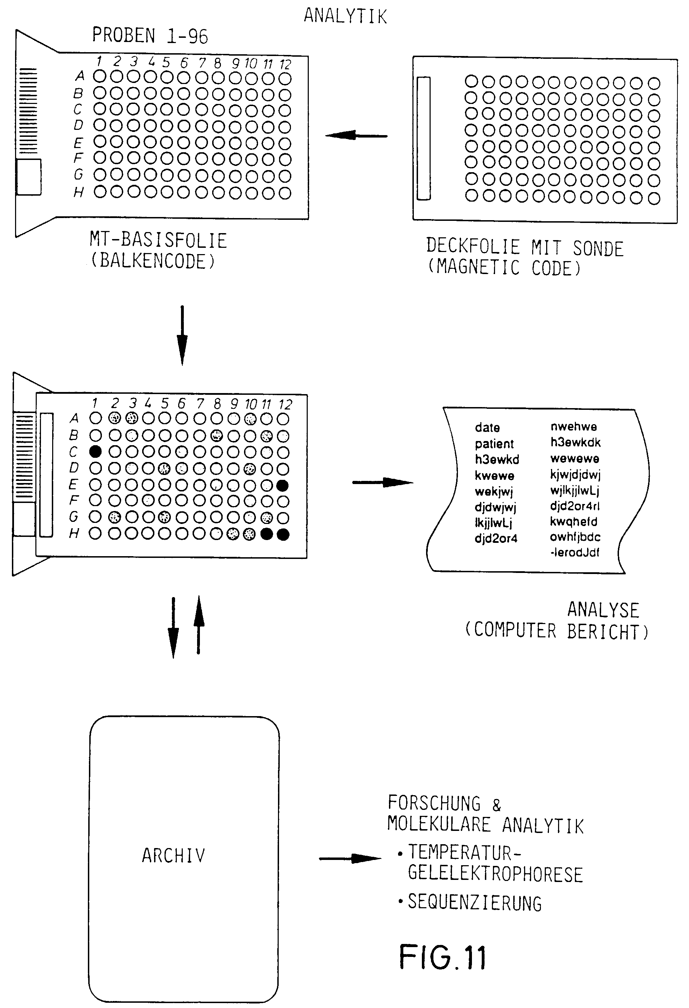

Die Figur 11 zeigt schematisch eine mögliche Ausführungsform des erfindungsgemäßen Verfahrens für die automatisierte Analyse von Großserien. Zum Testkit gehören die Folienbestandteile mit den lagerfähigen und gebrauchsfertigen Reagenzien. Die Balkenkodierung erlaubt eine Definition des Assay-Typs sowie der Belegung der Positionen. Der beschreibbare Magnetstreifen steht dann den Proben-spezifischen Daten zur Verfügung. Die Testfolien können nach der Analyse ohne Öffnen entsorgt oder für eventuelle weitere Analysen archiviert werden.

### Figur 12

Die schematische Darstellung erläutert eine spezielle Ausführungsform des erfindungsgemäßen Verfahrens. Ein zur Amplifikation verwendeter Primer ist oberflächenfixiert, z.B. an magnetischen Partikeln (magneto beads). Magneto beads lassen sich zu Zwecken der Amplifikation und Hybridisierung mit Hilfe eines Magnetfeldes in Form einer Suspension halten, zum Zwecke der Laser-Fluoreszenz-Beobachtung während des Temperaturgradienten-induzierten Dissoziationsprozesses jedoch der Lösung entziehen und an einem definierten Ort fixieren. So läßt sich der Laserstrahl direkt auf die Partikeloberfläche richten und gezielt die Fluoreszenz beim Abdissoziieren der Sonde verfolgen. Dieser Prozess gelingt z. B. bei Verwendung einer einzigen Schmelzdomäne und ermöglicht den Einsatz oligomerer, nicht-interkalierender Fluoreszenzfarbstoffe als optische Marker.

Reaktion und Analyse können in einem einzigen Reaktionskompartiment durchgeführt werden. Vorzugsweise wird ein Mikrotiter-Format gewählt, das es erlaubt, simultan 96 Proben oder Anteile von 96 Proben (8er oder 16er Strips) einer Analytik zu unterziehen. Das Reaktionsgefäß ist so konstruiert, daß es vorzugsweise Volumina von 20 - 100 µl trägt. Anstelle einzelner Reaktionsgefäße werden vorzugsweise Folien verwendet, die Vertiefungen oder Ausbuchtungen aufweisen, in denen die Proben aufgenommen werden. An sich bekannte Folien (Europäische Patente 0 442 942, 0 444 144, 0 539 369, 0 539 367, 0 539 368), eignen sich für das erfindungsgemäße Verfahren insbesondere, da sie besonders effizient thermostatierbar sind, billig in der Herstellung und problemlos in der umweltverträglichen Entsorgung. Die Verwendung optisch klarer Folien für den Bereich des sichtbaren Lichtes erlaubt eine On-Line-Registrierung von Fluoreszenz-Signalen handelsüblicher Fluroeszenz-Farbstoffe.

Die Folien lassen sich mit den allgemein benötigten Reagenzien befüllen (Enzyme, Primer, Puffer, Stabilisatoren etc.) und im lyophilisierten Zustand über lange Zeiträume konservieren. Als Stabilisatoren werden vorzugsweise Trehalose oder Saccharose eingesetzt. Die seriell angeordneten Reaktionsgefäße lassen sich nach Hinzufügen der zu analysierenden Proben hermetisch verschließen. Dieses kann durch Verschmelzen einer Deckfolie mit der Reaktionskompartiment-tragenden Folie geschehen. Es lassen sich auch solche Folien verwerten, die mit einem thermoplastischen Polymer beschichtet sind und sich unterhalb der Schmelztemperatur der eigentlichen Trägerfolien verschweißen lassen. Im Deckelbereich der Trägerfolie, oberhalb der Reaktionskompartimente, lassen sich Reagenzien fixieren oder kompartimentieren, die nicht von Anfang an am Reaktionsgeschehen teilnehmen sollen. Dies ist im erfindungsgemäßen Falle z. B. die spezifisch markierte Sonde, die in einem Puffergemisch lyophilisiert und stabilisiert wird, das nach erfolgter Amplifikationsreaktion für die Hybridisierung vom Amplifikationsprodukt und markierter Sonde benötigt wird. Nach Verschweißen der Reaktionskompartimente findet die Amplifikationsreaktion bei homogener Temperatur (3 SR, Self-sustained Sequence Replication; TAS, Transcription based amplification system) (J.C. Guatelli et al. (1990) Proc. Natl. Acad. Sci. 87, 1874-1878; Kwoh, D.Y., Davis, G.R., Whitfield, K.M., Chapelle, H.L., Dimichele, L.J. & Gingeras, T.R. (1989) Proc. Natl. Acad. Sci. USA 86, 1173-1177) oder im Thermocycler als Polymerase-Kettenreaktion (PCR) statt. In diesem Schritt werden Standard und Template im konstanten Verhältnis amplifiziert, so daß das Reaktionsendprodukt ca. 100 ng bis 1 µg amplifizierte Nukleinsäure enthält.

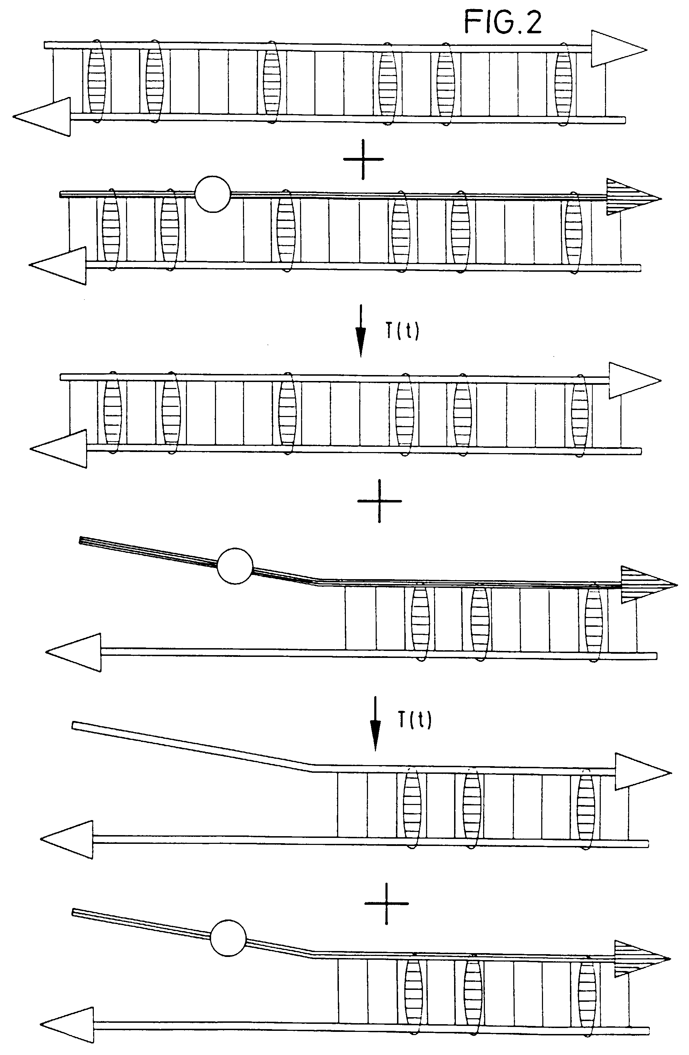

In einer möglichen, technisch sehr einfachen Ausführungsform (Figur 2), befindet sich im Reaktionskompartiment ein gelöster Lumineszenz-, vorzugsweise ein Fluoreszenzfarbstoff, vorzugsweise mit interkalierenden Eigenschaften (siehe unten), der an mehreren Positionen in doppelhelikalen Strukturen bindet und im gebundenen Zustand veränderte spektroskopische Eigenschaften besitzt. Wenn bei der späteren Analyse im zeitlichen Temperaturgradienten die entsprechenden Doppelstrangstrukturen denaturiert werden, wird der Farbstoff wieder freigesetzt. Dieser Vorgang wird spektroskopisch registriert. Eine notwendige Bedingung für diese Verfahrensweise ist jedoch, die Konzentration des freien Farbstoffes so zu wählen, daß sie größer ist als die Anzahl der freien Bindungsplätze. Andererseits führt eine Freisetzung des Farbstoffes aus einer Doppelstrangregion zur Bindung in einer anderen Struktur, ohne Änderung des Fluoreszenzsignales.

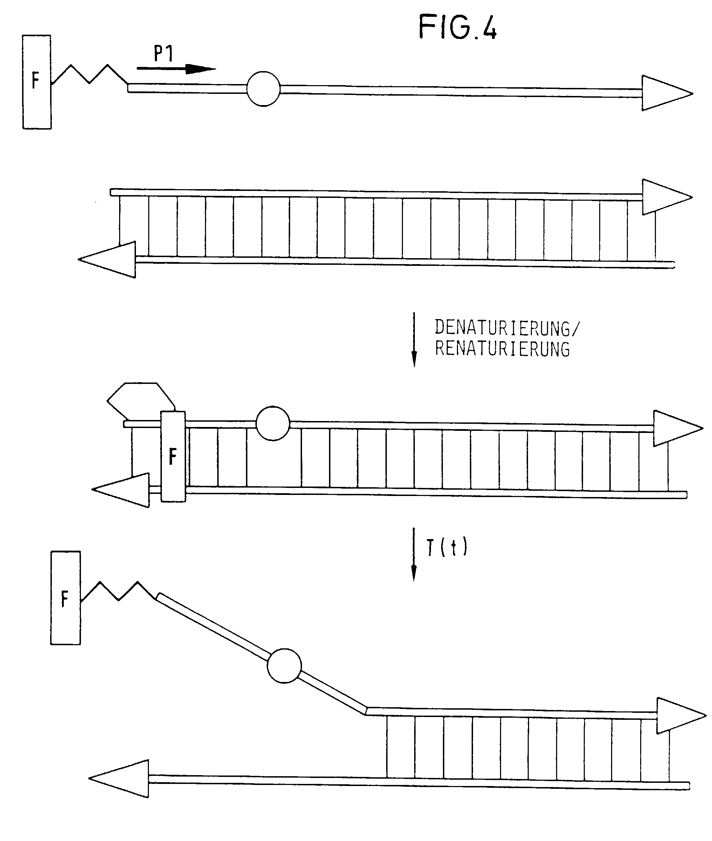

In einer weiteren bevorzugten Ausführung wird die wäßrige Lösung des Reaktionskompartimentes mit einer vorzugsweise an der Verschlußfolie kompartimentiert gelagerten Fluoreszenzfarbstoff-markierten Sonde unter Lösen der Sonde in Kontakt gebracht (Figuren 3 und 4). Nach Denaturierung und Renaturierung wird die Sonde fluoreszenzspektroskopisch verfolgt. Bei tiefen Temperaturen befindet sich die Sonde im Doppelstrang-Hybrid mit amplifiziertem Standard (Homoduplex) und amplifiziertem Template (Heteroduplex). Nun wird das Reaktionskompartiment zeitlich, vorzugsweise linear aufgeheizt, wobei zunächst der Heteroduplex partiell oder vollständig denaturiert und anschließend der Homoduplex partiell oder vollständig denaturiert. Aus dem relativen Verhältnis der Denaturierungssignale, gemessen über die Stufen der Fluoreszenzabnahmen (Figuren 9a bis 9e), läßt sich exakt der Template-Titer kalkulieren.

Als Denaturierungssignal wird vorzugsweise ein Fluoreszenzmarker verwandt. Insbesondere eignen sich hierzu Fluoreszenzfarbstoffe, die die Eigenschaft besitzen, nur dann stark zu fluoreszieren, wenn sie zwischen den Basenpaaren eingelagert werden (Interkalation). Wenn solche Farbstoffe aus einer Doppelhelix infolge eines Denaturierungsprozesses freigesetzt werden, so läßt sich das an einer Veränderung der Fluoreszenzintensität registrieren (Fluoreszenzabfall). Haben Doppelhelices unterschiedliche Stabilität wie im Falle von Homoduplex und Heteroduplex, dann finden diese Signaländerungen bei unterschiedlichen Temperaturen statt und lassen sich getrennt analysieren und auswerten. Die exakte Denaturierungstemperatur reflektiert darüberhinaus mögliche Sequenzunterschiede als Indikator für sogenannte Virusdrifts, die infolge von Mutation auftreten können.

Interkalierende Farbstoffe besitzen gelegentlich eine weitere günstige Eigenschaft, die sich auf die Halbwertzeit des angeregten Zustandes bezieht. Im Falle von Ethidiumbromid ist die Lebensdauer des Anregungszustandes mehr als 10 x länger, wenn sich das Fluoreszenzmolekül im interkalierten Zustand befindet. Dadurch läßt sich eine getaktete Laseranregung verwenden, wobei die Fluoreszenzintensität in der nachfolgenden Phase der Fluoreszenzemission ohne Streulicht-Einflüsse des Anregungslichtes gemessen werden können. Es ist mit dem erfindungsgemäßen Verfahren möglich, Farbstoffe wie Ethidiumbromid vorzugsweise in niedriger Konzentration (vorzugsweise 10⁻¹⁰ bis 10⁻⁷ M) zu verwenden.

Außer der direkten Messung einer Fluoreszenzintensität läßt sich auch erfindungsgemäß ein sogenannter Förster-Transfer (Energy-Transfer) zwischen eng benachbarten Fluorophoren oder der Parameter der Fluoreszenzpolarisation verwenden.

Eine hohe Spezifität des erfindungsgemäßen Verfahrens wird erzielt, wenn eine Sonde verwendet wird, die kovalent mit einem oder mehreren Farbstoffmolekülen verknüpft ist (Figur 4). Dies wird vorzugsweise dadurch erreicht, daß ein Primer zur Herstellung der Sonde verwendet wird, der endständig oder intern mindestens ein Farbstoffmolekül trägt. Nur wenn sich die Sonde im Zustand der Basenpaarung des Homoduplex oder Heteroduplex befindet, erhält man eine maximale Fluoreszenzintensität des Doppelstranginterkalierten Fluoreszenzfarbstoffes. Die Fluoreszenzintensität der parallel geführten Reaktionsansätze kann simultan mit Hilfe einer Kamera erfaßt werden. Die Einzelauswertung der Kanäle ergibt einen Fluoreszenzverlauf, wie er typisch in Figur 9 dargestellt ist. Die relative Höhe der Fluoreszenzänderungen kann über einen On-Line angeschlossenen Computer direkt in Kopienzahlen umgerechnet werden.

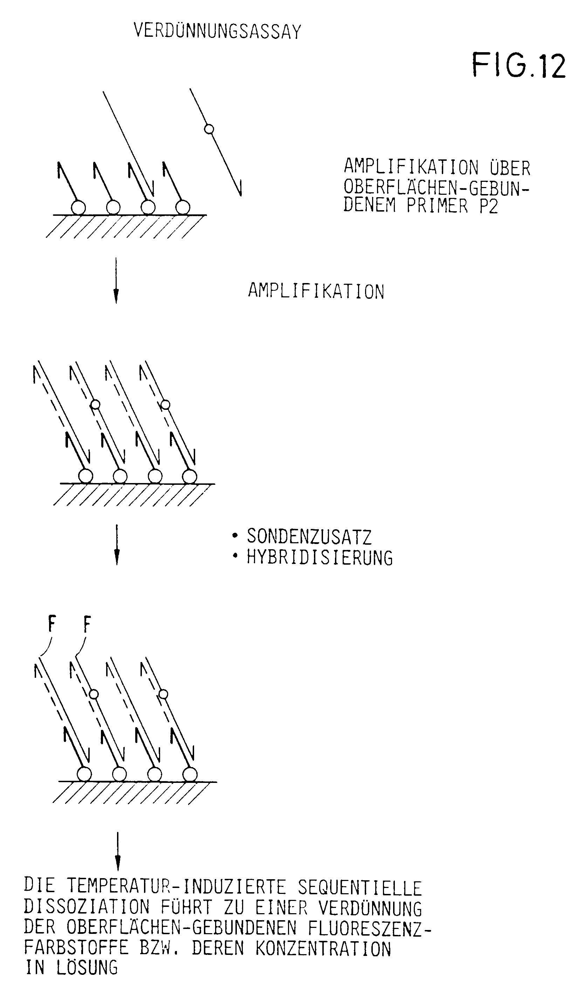

In einer weiteren bevorzugten Ausführungsform des erfindungsgemäßen Verfahrens wird die Reaktion innerhalb des Kompartimentes teilweise Festphasenträger-gekoppelt durchgeführt (Figur 12). Der Vorteil dieser Vorgehensweise ist darin begründet, daß sich mit Hilfe einer Laser-Optik gezielt die Oberfläche eines Festphasenträgers, oder alternativ die Lösung ohne den Festphasenträger, vermessen läßt. Das bedeutet für die praktische Durchführung, daß ein Fluoreszenzfarbstoff Verwendung finden kann, der nicht notwendigerweise seine meßbaren Parameter über Interkalation verändert. Somit können Sonden mit beliebigen, oligomeren Farbstoffen verwendet werden, mit denen eine mehr als zehnfache Steigerung der Fluoreszenzintensität erreicht werden kann. Der thermisch induzierte Denaturierungsprozess (siehe oben) kann dann verfolgt werden, indem die Dissoziation der Sonde von oberflächen-gebundenen, amplifizierten Nukleinsäuren (Probe und Standard) in die freie Lösung über die Abnahme der Fluoreszenz gemessen wird (Verdünnungseffekt). Dieses Vorgehen ist auf solche Analysen beschränkt, bei denen die zur Standardisierung herangezogene Doppelstrangregion in der thermodynamisch stabilsten Schmelzregion der Nukleinsäure liegt, oder bei Sonden, die nur eine Schmelzregion aufweisen.

Auf einen Zusatz einer markierten Sonde nach erfolgter Amplifikation kann verzichtet werden, wenn die Sonde aufgrund bestimmter Eigenschaften und Prozessführung der Amplifikation nicht am Amplifikationsgeschehen teilnehmen kann. Das kann erfindungsgemäß erreicht werden, wenn die Sonde in einer thermodynamisch stabilen Doppelstrangstruktur vorliegt, die während des Amplifikationsprozesses stabil bleibt und nicht am Reaktionsgeschehen teilnimmt. Es lassen sich z.B. markierte RNA-Doppelhelices hoher thermodynamischer Stabilität oder chemisch modifizierte Sonden einsetzen. Auf diese Weise läßt sich der Amplifikationsprozess so steuern, daß die Sonde bei der Amplifikationsreaktion immer doppelsträngig bleibt und am Amplifikationsgeschehen nicht teilnimmt, sei es, daß die Denaturierungstemperatur nicht ausreichend ist oder die beteiligten Enzyme doppelsträngige RNA oder die modifizierte Sonde nicht amplifizieren.

Amplifikationstechniken bergen die große Gefahr in sich, daß nach erfolgter Amplifikation, amplifizierte DNA- oder RNA-Moleküle in die Umgebung austreten können. Besondere Gefahr ist immer durch Aerosolbildung gegeben, die technisch nur schwer zu beherrschen ist. Das erfindungsgemäße Verfahren erlaubt es jedoch in seiner besonderen Ausführung, Amplifikationsreaktionen und Analytik am hermetisch geschlossenen Reaktionsgefäß durchzuführen und dieses anschließend im geschlossenen Zustand zu entsorgen. Damit wird ein ganz entscheidender Beitrag zur molekularen Laborhygiene und für die Sicherheit der Ergebnisse einer Routinediagnostik geleistet.

Eine Routinediagnostik ist unmittelbar mit der Notwendigkeit einer Archivierung der Ergebnisse und - nach Möglichkeit - der analysierten Proben verbunden. Das erfindungsgemäße Verfahren bietet hier wiederum eine nahezu ideale Möglichkeit:
- Die Folien lassen sich mit Test-spezifischer Strichmarkierung ausstatten und somit bezüglich Zieltest, Herstelldatum, Verfallsdatum etc. charakterisieren und datenmäßig erfassen.
- Die Folien lassen sich im hermetisch verschlossenen Zustand über lange Zeiräume archiviert lagern.
- Analysen lassen sich zu späteren Zeitpunkten wiederholen und überprüfen.
- Liefert die erfindungsgemäße Analysenmethode Indizien für das Vorliegen neuer interessanter DNA/RNA-Varianten, läßt sich das Probengemisch entnehmen und direkt auf dem flächigen Temperatur-Gelelektrophorese-Trennsystem präparativ auftrennen und z.B. einer Sequenzanalyse unterziehen.

Die Temperaturgradienten-Gelelektrophorese hat für kleinere Serien ihre Zuverlässigkeit für eine quantitative Amplifikationsanalytik gezeigt. Bei dieser Technik werden vorzugsweise solche PCR-Amplifikate verwendet, die G:C-reiche, primer-kodierte Sequenzen am stabileren Fragmentende tragen. Diese sogenannten G:C-Klammern garantieren den für eine Temperatur-Gelelektrophorese-Analyse notwendigen reversiblen Schmelzverlauf und unterdrücken eine vorschnelle Dissoziation der Fragmente in die Einzelstränge. Für eine Vielzahl von Analysen mit temperaturabhängiger Gelelektrophorese werden G:C-Klammern, die kostspielig im Einsatz sind und bei der Amplifikation Schwierigkeiten verursachen können, nicht mehr erforderlich sein. Die sequentielle Freisetzung der Fluoreszenzfarbstoffe in einem Assay kann durchaus irreversibel erfolgen, ohne daß das Ergebnis verfälscht wird.

Das erfindungsgemäße Verfahren hat beträchtliche wirtschaftliche Bedeutung, sowohl für die wirtschaftlichere Herstellung von Testkits, als auch in Bezug auf die Durchführungskosten der Analytik. Die Durchführung ist fast vollständig automatisierbar, wobei das Ergebnis der Analyse in Form eines Hard-Copy-Berichtes ausdruckbar ist. Damit ist die TESGA-Technik nicht nur auf den Einsatz für kostspielige, quantifizierende Analysen großer Wertschöpfung beschränkt, sondern auch einsetzbar für preiswerte Analysen. Das betrifft z.B. den Bereich der Mikrobiologie, Humangenetik, Phytoanalytik, forensische Analytik und die industrielle Forschung wie z.B. die Wirkstofforschung, das heißt Reihenuntersuchungen von Zielsubstanzen auf Wirkstoffe, die über DNA- oder RNA-Amplifikation oder -Modifikation bewertet werden können bis hin zu einfachen Toxizitätstests.

Das erfindungsgemäße Verfahren mit seinen vorteilhaften Eigenschaften wie
- PCR/3SR/TAS-Amplifikationseffizienz
- Eignung für Reihen- und Einzeluntersuchungen
- On-Line-Registrierung
- simultane Nukleinsäuren - Qualifizierung/Quantifizierung
- Eintopf-Mehrkomponenten Analyse
- Wegfall der Kontaminationsgefahr durch Amplifikate
- Niedrigkosten der Testreagenzien und Testdurchführung
- allgemein gültige - keine Test-spezifischen - Verfahrensprotokolle
erlaubt neue Einsatzmöglichkeiten der Amplifikationstechniken wie PCR, 3SR oder TAS.

In der genetischen Analytik wird auf längere Sicht ein Screening-Programm angestrebt, mit dem es beispielsweise möglich ist, aus Spuren von Biopsie-Material oder Fruchtwasserproben schwere genetische Erkrankungen nachzuweisen. Die Kostenbelastung für großflächige Programme ist hierbei ebenso eine entscheidende Restriktion wie die Tatsache, daß nur in den seltensten Fällen eine Analyse eines einzigen Genlocus ausreichend ist, Aussagen über das Überträgerpotential zuzulassen. Für die häufigste letale Erberkrankung in der kaukasischen Bevölkerung, der Cystischen Fibrose, sind neben der zunächst gefundenen Mutation (Delta 508) mehr als 18 weitere Mutationen beschrieben worden, die die Mukoviscidose auslösen können. Das erfindungsgemäße Verfahren erlaubt bei niedrigem zusätzlichen Kostenaufwand, eine große Anzahl von loci parallel zu analysieren und bei Bedarf - ohne großen Zusatzaufwand - weitere Testpositionen hinzuzunehmen. Das erfindungsgemäße Verfahren erlaubt beispielsweise auch die Untersuchung von Punktmutationen, die für bestimmte genetische Erkrankungen verantwortlich sind. Solche Erkrankungen können bislang nur mit der sehr schwer handhabbaren Allel-spezifischen Oligonukleotid-Hybridisierungstechnik (ASO) diagnostiziert werden.

Ähnlich bedeutungsvoll ist die Technologie für Thalassämie Screenings.

Damit wird deutlich, daß das erfindungsgemäße Verfahren auch die bisherigen Methoden zum Aufspüren von definierten Mutationen vereinfachen und erheblich kostengünstiger gestalten kann. Anstelle einer künstlich eingeführten Mutation, kann zur Normierung oder Standardisierung die gesuchte, natürlich auftretende Mutante eingesetzt werden, bzw. als Sonde verwandt werden. Somit kommen zur Durchführung des erfindungsgemäßen Verfahrens alle diejenigen Assays in Betracht, die z.B. mit differentiellen Oligonukleotid-Hybridisierungen als Filterassays durchgeführt werden.

Epidemiologische Studien bei Infektions- und Erberkrankungen werden für die internationale Medizin zu einem immer wichtigeren Aufgabengebiet. Erinnert sei an die beängstigende Ausbreitungsgeschwindigkeit moderner viraler Erkrankungen wie AIDS (HIV), bedingt durch die veränderten soziobiologischen Strukturen. Eine entscheidende Voraussetzung für Vaccinierungs- und Therapieansätze ist eine möglichst umfassende und sorgfältige epidemiologische Erfassung des Ist-Zustandes mit epidemiologisch-prognostischer Bedeutung. Dies betrifft nicht nur das Auftreten des Virus selbst, sondern auch die geografische Verteilung seines Variantenspektrums mit einer datenmäßigen Erfassung der Krankheitssymptomatik. Solche Studien werden erst durch eine aussagefähige automatisierte Analytik wirtschaftlich vertretbar.

Die Pharmaforschung stößt seit geraumer Zeit an die Grenzen der Machbarkeit auf ihren angestammten Aktivitätsfeldern. Es gibt viele Versuche, den offensichtlichen Beschränkungen herkömmlicher Pharmaforschung durch neue Konzeptionen zu begegnen. Angeführt sei das Beispiel des strategisch-intelligenten Wirkstoffdesign, das es erlauben soll, auf der Basis fundierten Wissens über eine Zielstruktur wie der eines Rezeptors, Effektormoleküle mit vorberechneter Struktur gezielt zu synthetisieren und somit herkömmliche Screening Methoden zu ersetzen. Eine zweite zukunftsträchtige Strategie ist die evolutive Biotechnologie mit ihrem Potential, unter Ausnutzung evolutiver Systeme, Stoffe mit erwünschtem Wirkpotential zu generieren. In der Pharmaforschung der Zukunft müssen aus Kostengründen als auch projektbedingt personalintensive, randomisierte Syntheseprogramme vermieden werden.

Das erfindungsgemäße Verfahren kann hierbei einen wichtigen Beitrag leisten. Die Tauglichkeit für Reihenuntersuchungen mit quantifizierenden DNA/RNA-Tests bei niedrigen Kosten erlaubt es, neue Tests für automatisiertes Aufspüren von Wirkstoffen in der Onkologie, bei viralen und bakteriellen Infektionen sowie Tests für toxikologische Studien aufzubauen. Es werden neue Assay-Systeme in Kombination mit zellulären in vitro-Systemen denkbar, die mehr und mehr Tiermodelle ersetzen werden. Dabei darf auch nicht vergessen werden, daß Kultivierungs- und Testzeiten erheblich verkürzt werden, da z. B. Variationen auf dem mRNA-Level unmittelbar erfaßt werden, ohne daß langwierig zelluläre Folgereaktionen gemessen werden müssen. Für eine Reihe von Assays könnte es möglich werden, transformierte Zellkulturen durch Primärkulturen z. B. aus Blut zu ersetzen. Die gleichzeitige hohe Sensitivität würde es auch erlauben, Differenzierungsparameter zu registrieren, um Zelltypspezifische Wirkungen zu messen. Gedacht ist z.B. an bestimmte Leukozyten-Subpopulationen (Makrophagen, T4-Zellen etc.) mit ihren charakteristischen Rezeptorfunktionen und Infizierbarkeiten mit Viren wie HIV.

Biologische Experimente mit rekombinanten Systemen, insbesondere Freisetzungsexperimente verlangen sowohl von wissenschaftlicher Seite als auch von seiten des Umweltschutzes nach einer sorgfältigen Analytik von Genen, der Erfassung der Genpersistenz in Populationen, Genveränderungen und Genaktivitäten. Als Beispiel seien antivirale Therapien bei Pflanzen genannt, bei denen versucht wird, Virusresistenz gegen bestimmte Viren dadurch zu erzeugen, daß transgene Pflanzen konstruiert werden, die virale Hüllproteine erzeugen und so die Pflanzen vor Befall mit einem Fremdvirus schützen. Ein Freisetzungsexperiment oder ein Einkreuzen in natürlich vorkommende Populationen setzt ein genaues Wissen voraus, zum Beispiel:
- Wie ist das Segregationsverhalten der rekombinanten Pflanzen bezüglich des rekombinanten Locus?
- Wie aktiv sind die Hüllprotein-Gene bezogen auf die Population und die Generationszahl?
- Wie reagiert der virale Zielorganismus auf den neuen Selektionsstreß?

Das erfindungsgemäße Verfahren ermöglicht hier Reihenuntersuchungen und insbesondere die Erfassung von Probenkollektiven in einem einzigen Test, um quantitative Aussagen über Populationen zu erhalten. Gleichzeitig werden Drift-Phänomene bei Resistenzbildung verfolgbar.

Mikrobiologische Tests der Bakteriologie werden traditionsgemäß durch Kultivierung oder direkte Dot-Hybridisierung durchgeführt. Solche Tests waren bislang durch aussagefähigere Tests kaum zu verdrängen, allein bedingt durch den Kostenaspekt. Das erfindungsgemäße Verfahren kann diese Situation vollständig verändern. Es stellt bei diesem Verfahren keinen entscheidenden Kostenfaktor dar, wenn eine Probe simultan auf mehrere Erreger gleichzeitig getestet werden muß oder wenn ein Erreger in einem Antibiogramm analysiert werden muß.

Der letzgenannte Analysentyp kann insbesondere bei virologischen Analysen eine große Rolle spielen, wenn Virusresistenzen ausgetestet werden müssen bzw. quantifizierend erfaßt werden müssen. Für die Virologie ist das erfindungsgemäße Verfahren vorteilhaft, da es eine stark vereinfachte Analytik durch eine kostengünstige und aufwandreduzierte Kopplung von Anzucht- und Testverfahren einer Viruspropagation erlaubt.

In der Nahrungsmittelindustrie wurden in erster Linie umfangreiche Kontrollen zur Untersuchung von einigen bestimmten mikrobiologischen Erregerklassen durchgeführt, jedoch ausgehend von einer Vielzahl von Probenmaterialien der Ausgangsprodukte, der Verfahrensschritte, des apparativen Containments und der Endprodukte. Ähnlich wie in der Pharmaindustrie werden bei der Verarbeitung Großansätze von erheblichem ökonomischen Wert bearbeitet. Die Minimierung der Analysendauer verbunden mit einer zuverlässigen Testaussage stellt hierbei einen kritischen Faktor dar. Zum Beispiel erfordern Salmonellentests häufig langwierige Anzüchtungsverfahren der Krankheitserreger. Das erfindungsgemäße Verfahren bewirkt mit seinen auch quantitativen Aussagen in Reihenuntersuchungen einen Verzicht auf Anzuchtverfahren und erspart kostspielige Prozessführungsschritte und Lagerungen.

Phytopathologische Analysen sind oft prohibitiv teuer. Selbst wenn in der Regel nur repräsentative Probenkollektive von Saatgut oder Zier- und Nutzpflanzen der Analyse beispielsweise auf Virusbefall zugeführt werden, so ließ die Anzahl der Proben aus Kostengründen bislang fast ausschließlich immunologische ELISA-Verfahren zu, die häufig nicht oder nur unzureichend funktionieren. Die Phytoanalytik bedarf also anderer Analysenkonzepte, um dem Bedarf gerecht zu werden. Diese Konzepte werden durch das erfindungsgemäße Verfahren bereitgestellt.

Nachdem nun erstmals RNA-Parameter gefunden worden sind, die eine exakte Wald-Schadensanalyse zulassen, läßt sich mit dem erfindungsgemäßen Verfahren ein umfangreiches Programm zur Erfassung und der zeitlichen und geographischen Ausbreitung von Waldschäden konzipieren.

Die Molekularbiologische Forschung bemüht sich in steigendem Maße um Analysenverfahren, die Serientauglichkeit haben, um beispielsweise das sogenannte HUGO-Projekt zur vollständigen Sequenzaufklärung des humanen Genoms sowie der Genome anderer wichtiger Organismen erfolgreich durchführen zu können. In Zukunft wird es bei diesem Projekt nicht nur darum gehen, ein singuläres Genom vollständig zu analysieren, sondern auch bestimmte Loci mit krankheitsbezogenem Potential zu analysieren, wie es vergleichbar bei Identitätsuntersuchungen notwendig ist (HLA-Analytik, Haplotyp-Assoziation, genetische Variabilität etc.).

Das erfindungsgemäße Verfahren ermöglicht auf DNA Ebene (amplifizierte loci, Gendosis) und auf mRNA Ebene Expressionsoptimierungen, Promoterkontrolle etc. zu verfolgen und im Screening von Mutageneseverfahren einzusetzen. Dieses Verfahren erweist sich hierbei als eine wichtige Komplementärtechnik zum "cell sorting".

## Patentansprüche

1. Verfahren zur qualitativen und quantitativen Bestimmung von mindestens einer in vitro-amplifizierten Nukleinsäure in einem verschlossenen Reaktionsraum,
- wobei während oder nach der Amplifizierung der Nukleinsäure mindestens eine Sonde vorhanden ist, die mit der nachzuweisenden Nukleinsäure in Wechselwirkung tritt,
- wobei spektroskopisch meßbare Parameter der Sonde eine Änderung erfahren und dabei ein meßbares Signal erzeugt wird,
- die zu messende Probe der Einwirkung eines Gradienten ausgesetzt wird, der Nukleinsäuren mindestens teilweise denaturieren kann
- unter Erfassung des sich durch die Einwirkung des Gradienten ändernden meßbaren Parameters und
- die gesamte Amplifikationsreaktion einschließlich der qualitativen und quantitativen Bestimmung in einem verschlossenen Reaktionsraum ohne zwischenzeitliche Öffnung durchgeführt wird.

2. Verfahren gemäß Anspruch 1, wobei die Sonde, deren spektroskopisch meßbarer Parameter mindestens ein Lumineszenz- oder Fluoreszenzfarbstoff ist, einen Nukleinsäureanteil enthält und dessen Wechselwirkung mit den in vitro-amplifizierten Nukleinsäuren in Abhängigkeit des Denaturierungszustandes mit einer Änderung des spektroskopischen Meßsignals einhergeht, vorzugsweise durch Interkalation des Farbstoffes in die Nukleinsäuredoppelhelix oder durch Verdünnungs- oder Konzentrierungseffekt in dem Reaktionsraum.

3. Verfahren gemäß mindestens einem der Ansprüche 1 oder 2, wobei ein Denaturierungsprozess über eine Änderung der Wellenlänge und/oder einen Lumineszenz bzw. Fluoreszenzintensitätsshift und/oder Fluoreszenzpolarisationsänderung und/oder eine Änderung der Lebenszeit eines angeregten Zustandes oder über das Prinzip des sogenannten "Energy Transfer" oder über einen Konzentrationseffekt erfaßt wird.

4. Verfahren gemäß mindestens einem der Ansprüche 1 bis 3, dadurch gekennzeichnet, daß in einem Reaktionsraum mehrere, spektroskopisch voneinander unterscheidbare Farbstoffe eingesetzt werden, mit denen sich verschiedene amplifizierte Nukleinsäuren analysieren lassen und/oder über die mindestens eine unabhängige Eichsubstanz einführbar ist.

5. Verfahren gemäß mindestens einem der Ansprüche 1 bis 4, dadurch gekennzeichnet, daß die Lumineszenz, insbesondere Fluoreszenz der Farbstoffe durch einen Laser kontinuierlich oder getaktet angeregt wird.

6. Verfahren gemäß mindestens einem der Ansprüche 1 bis 5, dadurch gekennzeichnet, daß die amplifizierten Nukleinsäuren mindestens einen koamplifizierten Nukleinsäure-Standard enthalten, dessen Sequenz zu einer zu bestimmenden Sequenz homolog ist, vorzugsweise identisch mit Ausnahme mindestens einer Punktmutation, die vorzugsweise in einem Sequenzbereich niedrigster Stabilität liegt, jedoch außerhalb der primer-Bindungsstellen bei enzymatischer Amplifikation.

7. Verfahren gemäß mindestens einem der Ansprüche 1 bis 5, dadurch gekennzeichnet, daß die amplifizierten Nukleinsäuren mindestens einen koamplifizierten Nukleinsäurestandard enthalten, dessen Sequenz mindestens in den Primer-Regionen absolut homolog ist, ansonsten jedoch die Sequenz des Nukleinsäurestandards von der Sequenz der amplifizierten Nukleinsäure in mehr als einer Position abweicht.

8. Verfahren gemäß mindestens einem der Ansprüche 1 bis 7, dadurch gekennzeichnet, daß der Nukleinsäure Standard teilweise selbst ein natürlicher Bestandteil der zu analysierenden Nukleinsäure ist.

9. Verfahren gemäß mindestens einem der Ansprüche 1 bis 8, dadurch gekennzeichnet, daß die Amplifikation in homogener Phase oder mit einem Festphasenträger gekoppelten Primer durchgeführt wird, an dessen verlängerter Sequenz die markierte Sonde hybridisieren kann und deren Konzentration und/oder Konformation somit entweder gezielt auf dem Festphasenträger oder in der freien Lösung bestimmt werden kann.

10. Verfahren gemäß mindestens einem der Ansprüche 1 bis 9, dadurch gekennzeichnet, daß mindestens ein Fluoreszenzfarbstoff an ein Nukleinsäuremolekül gekoppelt ist, dessen Sequenz identisch oder homolog mit der nachzuweisenden Nukleinsäure oder dem koamplifizierten Nukleinsäure-Standard ist.

11. Verfahren gemäß mindestens einem der Ansprüche 1 bis 10, dadurch gekennzeichnet, daß das Fluoreszenzfarbstoff-gekoppelte Nukleinsäuremolekül dem Reaktionsgemisch nach erfolgter Amplifikation zugesetzt wird, wobei die Hybridisierung mit den amplifizierten Nukleinsäuren durch einen thermischen Denaturierungsschritt mit nachfolgendem Renaturierungsschritt erfolgt.

12. Verfahren gemäß mindestens einem der Ansprüche 1 bis 4 und 10, dadurch gekennzeichnet, daß das Fluoreszenzfarbstoff-gekoppelte Nukleinsäuremolekül dem Reaktionsgemisch vor erfolgter Amplifikation zugesetzt wird, wobei die Sonde als nicht amplifizierbare Doppelstrang-RNA oder als nicht amplifizierbare chemisch modifizierte Nukleinsäure zugesetzt wird.

13. Verfahren gemäß mindestens einem der Ansprüche 1 bis 12, dadurch gekennzeichnet, daß zur Amplifikation vorzugsweise ein Primer eines Primerpaares verwendet wird, der 5`-terminal eine G:C-reiche Region kodiert von vorzugsweise 15 bis 20 G:C - Resten.

14. Verfahren gemäß mindestens einem der Ansprüche 1 bis 10, dadurch gekennzeichnet, daß die zur Bestimmung verwendete Sonde ein Oligo- oder Polynukleotid ist, welches mindestens zwei chemische Strukturelemente aufweist, die in der Lage sind elektromagnetische Wellen zu absorbieren und/oder aufgrund einer Anregung zu emittieren und wobei eines der Strukturelemente durch Einfluß elektromagnetischer Wellen eine dauerhafte Bindung zu einer anderen Position des Oligo- oder Polynukleotids zu knüpfen oder zu lösen vermag.

15. Verfahren nach Anspruch 14, wobei das eine elektromagnetische Welle absorbierende und/oder emittierende Strukturelement ein chromophores System aufweist.

16. Verfahren nach Anspruch 15, wobei das chromophore System Farbstoffsubstituent luminesziert.

17. Verfahren nach mindestens einem der Ansprüche 14 bis 16, wobei das unter elektromagnetischem Einfluß dauerhafte Bindungen knüpfende oder lösende Strukturelement ein photochemischer Vernetzer ist.

18. Verfahren nach Anspruch 17, wobei der photochemische Vernetzer ein Psoralenderivat ist.

19. Verfahren nach mindestens einem der Ansprüche 14 bis 18, wobei der Abstand der Strukturelemente 8 bis 12, vorzugsweise 10, Nukleotidpositionen nicht unterschreiten darf.

20. Verfahren gemäß mindestens einem der Ansprüche 1 bis 19, dadurch gekennzeichnet, daß Foliensysteme mit Vertiefungen oder Ausbuchtungen als Reaktionsräume verwendet werden, die thermisch verschweißbar sind und als Aufnahme für verwendungsfertige Reagenziengemische in lyophilisierter oder Matrix-gebundener Form dienen, sowie zur direkten optischen Vermessung geeignet sind.

21. Verfahren gemäß mindestens einem der Ansprüche 1 bis 20, dadurch gekennzeichnet, daß die vorgelegten Reagenzien in räumlich getrennten Matrices gelagert werden und nach dem Schließen eines Reaktionsraumes zeitlich getrennt dem Reaktionsgeschehen zugeführt werden können.

22. Verfahren gemäß mindestens einem der Ansprüche 1 bis 21, dadurch gekennzeichnet, daß der Abstand der Reaktionsräume so gewählt ist, daß eine Auswertung mit kommerziellen Apparaturen, wie mikrotitrationsformatierten Geräten erfolgt.

23. Verfahren gemäß mindestens einem der Ansprüche 1 bis 22, dadurch gekennzeichnet, daß das Nukleinsäuregemisch zur Analyse einem zeitlich gesteuerten Temperaturgradienten unterworfen wird mit vorzugsweise linearem Verlauf, wobei die Änderung des spektroskopischen Parameters als Funktion der Zeit und/oder der Temperatur verfolgt wird.

24. Verfahren gemäß Anspruch 23, wobei die Analyse der Konformation des Nukleinsäuregemisches mit Hilfe der Temperaturgelelektrophorese, einem chromatographischen Verfahren oder direkt in homogener Lösung oder in Kombination dieser Verfahren erfolgt.

25. Verfahren gemäß Anspruch 23 und/oder 24, dadurch gekennzeichnet, daß aus der Abhängigkeit des optischen Parameters mit der Temperatur und/oder der Zeit auf die Anwesenheit und/oder Anzahl und/oder Homologie einer gesuchten Nukleinsäure zum korrespondierenden Standard geschlossen werden kann.

26. Verfahren gemäß mindestens einem der Ansprüche 1 bis 25, dadurch gekennzeichnet, daß die Auswertung der Daten on line durch ein Datenverarbeitungsystem erfolgt.

27. Verfahren gemäß mindestens einem der Ansprüche 1 bis 26, bei dem ein System von in Mikrotiter-Dimensionierung angeordneten Reaktionskompartimenten, vorzugsweise einem Foliensystem mit gebrauchsfertigen Reagenzien in lyophylisierter Form, eingesetzt wird.

28. Verfahren gemäß Anspruch 27, dadurch gekennzeichnet, daß die Reagenzien in mindestens einer wasserlöslichen Matrix fixiert und/oder gelagert werden.

29. Verfahren gemäß Anspruch 27 und/oder 28 , wobei die Matrix Stabilisatoren enthält, vorzugsweise Zucker, insbesondere Trehalose oder Saccharose.

30. Verfahren gemäß mindestens einem der Ansprüche 27 bis 29, wobei das Reaktionskompartiment und/oder weitere Reagenzienreservoire Amplifikationsprimer, Pufferbestandteile, mindestens eine Polymerase und Kofaktoren enthält.

31. Verfahren gemäß einem der Ansprüche 27 bis 30, wobei die Reaktionskompartiment-verschließende Folie mindestens ein weiteres Reagenzienreservoir in einer Matrix enthält, wobei vorzugsweise dort die markierte Sonde mit den für die Hybridisierung notwendigen Reagenzien gelagert ist.

32. Verwendung einer Oligo- oder Polynukleotid-Sonde mit mindestens einem chemischen Strukturelement, das in der Lage ist mit elektromagnetischen Wellen unter Lösen oder Knüpfen dauerhafter Bindungen und/oder durch Absorption oder Emission von Strahlung in Wechselwirkung zu treten und wobei dieses Strukturelement nicht ein Purin- oder Pyrimidin-Substituent der natürlich vorkommenden Nukleotidbausteine darstellt zur Durchführung des Verfahrens nach mindestens einem der Ansprüche 1 bis 31.

33. Verwendung nach Anspruch 32, wobei das dauerhafte Bindungen zu knüpfen vermögende Strukturelement Psoralen oder ein Psoralenderivat ist.

34. Verwendung nach einem der Ansprüche 32 und/oder 33, wobei mindestens eines der mit elektromagnetischer Strahlung in Wechselwirkung zu treten vermögende Strukturelement luminesziert.

35. Verwendung nach mindestens einem der Ansprüche 32 bis 34, wobei der Abstand der Strukturelemente des Oligo- oder Polynukleotids mindestens 8 bis 12 Nukleotide beträgt.

## Claims

1. A method for the qualitative detection and quantitative determination of at least one in-vitro amplified nucleic acid in a closed reaction chamber wherein
- at least one probe is present during or after the amplification of said nucleic acid which probe will interact with the nucleic acid to be detected;
- spectroscopically measurable parameters of said probe are changed, thereby generating a measurable signal;
- the sample to be measured is exposed to a gradient which is capable of at least partially denaturing nucleic acids;
- the measurable parameter which changes under the action of the gradient is recorded; and
- the whole amplification reaction including the qualitative detection and quantitative determination is performed in a closed reaction chamber with no intermediate opening thereof.

2. The method according to claim 1 wherein said spectroscopically measurable parameter of said probe is at least one luminescent or fluorescent dye and said probe contains a nucleic acid fraction the interaction of which with said in-vitro amplified nucleic acids generates a spectroscopical measuring signal which is changed depending on the denaturing condition, preferably by intercalation of the dye in the nucleic acid double helix or by dilution or concentration effects in the reaction chamber.

3. The method according to at least one of claims 1 or 2 wherein a denaturing process is detected through a change of the wavelength and/or a luminescence or fluorescence intensity shift and/or a fluorescence polarization change and/or a change of the lifetime of an excited state or through the so-called "energy transfer" principle or through a concentration effect.

4. The method according to at least one of claims 1 to 3, characterized in that several spectroscopically distinct dyes are employed in a reaction chamber by means of which different amplified nucleic acids can be analyzed and/or by means of which at least one independent calibration substance can be introduced.

5. The method according to at least one of claims 1 to 4, characterized in that the luminescence, especially fluorescence, of the dyes is excited by a laser in a continuous or pulsed mode.

6. The method according to at least one of claims 1 to 5, characterized in that said amplified nucleic acids contain at least one co-amplified nucleic acid standard the sequence of which is homologous to a sequence to be determined, preferably identical except at least one point mutation which is preferably positioned in a sequence region of lowest stability, but outside the primer binding sites in enzymatic amplification.

7. The method according to at least one of claims 1 to 5, characterized in that said amplified nucleic acids contain at least one co-amplified nucleic acid standard the sequence of which is absolutely homologous in the primer regions, but otherwise the sequence of said nucleic acid standard is different from the sequence of said amplified nucleic acid in more than one position.

8. The method according to at least one of claims 1 to 7, characterized in that said nucleic acid standard is partially itself a natural component part of the nucleic acid to be analyzed.

9. The method according to at least one of claims 1 to 8, characterized in that said amplification is performed in homogeneous phase or with a solid-phase support coupled primer to the extended sequence of which the labeled probe can hybridize and the concentration and/or conformation of which can thus be determined in a well-aimed manner either on said solid phase support or in the free solution.

10. The method according to at least one of claims 1 to 9, characterized in that at least one fluorescent dye is coupled to a nucleic acid molecule the sequence of which is identical with or homologous to the nucleic acid to be detected or said co-amplified nucleic acid standard.

11. The method according to at least one of claims 1 to 10, characterized in that said fluorescent-dye coupled nucleic acid molecule is added to the reaction mixture after the amplification has been accomplished, the hybridization with the amplified nucleic acids being effected by a thermal denaturing step followed by a renaturing step.

12. The method according to at least one of claims 1 to 4 and 10, characterized in that said fluorescent-dye coupled nucleic acid molecule is added to the reaction mixture prior to the amplification, the probe being added as a non-amplifiable double-stranded RNA or as a non-amplifiable chemically modified nucleic acid.

13. The method according to at least one of claims 1 to 12, characterized in that the amplification preferably employs a primer of a pair of primers which encodes a region rich in G:C of preferably 15 to 20 G:C residues at the 5' terminus.

14. The method according to at least one of claims 1 to 10, characterized in that the probe used for the determination is a oligo- or poly-nucleotide which has at least two chemical structural elements which are capable of absorbing electromagnetic waves and/or emitting them due to an excitation wherein one of said structural elements is capable of forming or cleaving a permanent bond to another position of said oligo- or poly-nucleotide under the action of electromagnetic waves.

15. The method according to claim 14 wherein said structural elements absorbing and/or emitting an electromagnetic wave comprises a chromophorous system.

16. The method according to claim 15 wherein said chromophorous system is a dye substituent and undergoes luminescence.

17. The method according to at least one of claims 14 to 16 wherein said structural element forming or cleaving permanent bonds under electromagnetic influence is a photochemical cross-linking agent.

18. The method according to claim 17 wherein said photochemical cross-linking agent is a psoralen derivative.

19. The method according to at least one of claims 14 to 18 wherein the distance of said structural elements must not be less than 8 to 12, preferably 10, nucleotide positions.

20. The method according to at least one of claims 1 to 19, characterized in that plastic sheet systems with recesses or protrusions are used as said reaction chambers which sheets are thermally sealable and serve as recipients for ready-to-use reagent mixtures in lyophilized or matrix-bound form and are suitable for direct optical measurement.

21. The method according to at least one of claims 1 to 20, characterized in that the reagents to be used are stored in spatially separated matrices and can be separately introduced into the reaction after the closing of a reaction chamber.

22. The method according to at least one of claims 1 to 21, characterized in that the distance between the reaction chambers is selected to allow processing by commercial devices, such as microtitration-format devices.

23. The method according to at least one of claims 1 to 22, characterized in that the nucleic acid mixture is exposed to a time-controlled temperature gradient for analysis which is preferably linear wherein the change of the spectrocopic parameter is followed as a function of time and/or temperature.

24. The method according to claim 23 wherein the analysis of the conformation of the nucleic acid mixture is performed by means of temperature gel electrophoresis, a chromatographic method or directly in homogeneous solution or by a combination of such methods.

25. The method according to claim 23 and/or 24, characterized in that the presence and/or the number and/or homology to the corresponding standard of a sought nucleic acid can be concluded from the nature of the dependence of the optical parameter on temperature and/or time.

26. The method according to at least one of claims 1 to 25, characterized in that the evaluation of the data is performed on-line by a data processing system.

27. The method according to at least one of claims 1 to 26 which employs a system of reaction compartments arranged in microtitration format, preferably a plastic sheet system with ready-to-use reagents in lyophilized form.

28. The method according to claim 27, characterized in that said reagents are fixed and/or stored in at least one water-soluble matrix.

29. The method according to claim 27 and/or 28 wherein said matrix contains stabilizers, preferably sugars, especially trehalose or sucrose.

30. The method according to at least one of claims 27 to 29 wherein said reaction compartment and/or additional reagent reservoirs contain amplification primers, buffer components, at least one polymerase, and cofactors.

31. The method according to any of claims 27 to 30 wherein the sheet which seals the reaction compartment contains at least one additional reagent reservoir within a matrix which is preferably the place where the labeled probe is stored together with the reagents necessary for hybridization.

32. Use of an oligo- or poly-nucleotide probe having at least one chemical structural element which is capable of interacting with electromagnetic waves to cleave or form permanent bonds and/or by the absorption or emission of radiation wherein said structural element is not a purine or pyrimidine substituent of the naturally occurring nucleotide building blocks, for performing the method according to at least one of claims 1 to 31.

33. The use according to claim 32 wherein said structural element capable of forming permanent bonds is psoralen or a psoralen derivative.

34. The use according to any of claims 32 and/or 33 wherein at least one said structural elements capable of interacting with electromagnetic radiation is luminescent.

35. The use according to at least one of claims 32 to 34 wherein the distance of said structural elements of the oligo- or polynucleotide is at least 8 to 12 nucleotides.

## Revendications

1. Procédé pour le dosage qualitatif et quantitatif d'au moins un acide nucléique amplifié in vitro, dans un espace de réaction fermé,
- dans lequel, pendant ou après l'amplification de l'acide nucléique, au moins une sonde est présente, qui entre en interaction avec l'acide nucléique à détecter,
- dans lequel les paramètres de la sonde, mesurables par spectroscopie, subissent une modification, ce qui produit un signal mesurable,
- l'échantillon à mesurer est exposé à l'action d'un gradient, qui peut au moins partiellement dénaturer les acides nucléiques,
- avec détection du paramètre mesurable, qui varie sous l'action du gradient, et
- la totalité de la réaction d'amplification, y compris le dosage qualitatif et quantitatif, est mise en oeuvre dans un espace de réaction fermé, sans ouverture intermédiaire.

2. Procédé selon la revendication 1, dans lequel la sonde, dont le paramètre mesurable par spectroscopie est au moins un colorant luminescent ou fluorescent, contient une certaine proportion d'acides nucléiques, et dont l'interaction avec les acides nucléiques amplifiés in vitro se produit en fonction de l'état de dénaturation, par une modification du signal de mesure spectroscopique, de préférence par intercalation du colorant dans la double hélice de l'acide nucléique, ou par un effet de dilution ou de concentration dans l'espace de réaction.

3. Procédé selon au moins l'une des revendications 1 ou 2, dans lequel un processus de dénaturation est détecté par une modification de la longueur d'onde et/ou par un déplacement de l'intensité de luminescence ou de fluorescence et/ou par une modification de la polarisation de fluorescence et/ou par une modification de la durée de vie d'un état excité, ou par le principe de ce que l'on appelle un "Energy Transfer" (transfert d'énergie), ou encore par un effet de concentration.

4. Procédé selon au moins l'une des revendications 1 à 3, caractérisé en ce qu'on utilise dans un espace de réaction plusieurs colorants, pouvant se distinguer les uns des autres par spectroscopie, à l'aide desquels il est possible d'analyser différents acides nucléiques amplifiés, et/ou par lesquels il est possible d'introduire au moins une substance étalon indépendante.

5. Procédé selon l'une des revendications 1 à 4, caractérisé en ce que la luminescence, en particulier la fluorescence, des colorants, est excitée en continu ou selon un rythme prédéfini par un laser.

6. Procédé selon au moins l'une des revendications 1 à 5, caractérisé en ce que les acides nucléiques amplifiés contiennent au moins un étalon d'acide nucléique co-amplifié, dont la séquence est homologue à une séquence à déterminer, de préférence identique, à l'exception d'au moins une mutation ponctuelle, qui de préférence se trouve dans une zone de séquence présentant la stabilité la plus faible, mais à l'extérieur des points de fixation de l'amorce lors d'une amplification enzymatique.

7. Procédé selon au moins l'une des revendications 1 à 5, caractérisé en ce que les acides nucléiques amplifiés contiennent au moins un étalon d'acide nucléique co-amplifié, dont la séquence présente une homologie absolue au moins dans les régions d'amorce, mais pour le reste la séquence de l'étalon d'acide nucléique s'écarte, sur plus d'une position, de la séquence de l'acide nucléique amplifié.

8. Procédé selon au moins l'une des revendications 1 à 7, caractérisé en ce que l'étalon d'acide nucléique est partiellement, par lui-même, un constituant naturel de l'acide nucléique à analyser.

9. Procédé selon au moins l'une des revendications 1 à 8, caractérisé en ce que l'amplification est mise en oeuvre en phase homogène ou avec une amorce couplée à un support en phase solide, amorce à la séquence prolongée de laquelle peut s'hybrider la sonde marquée, et dont la concentration et/ou la conformation peuvent ainsi être adaptées à volonté au support en phase solide, ou encore en solution libre.

10. Procédé selon au moins l'une des revendications 1 à 9, caractérisé en ce qu'au moins un colorant fluorescent est couplé à une molécule d'acide nucléique, dont la séquence est identique à celle de l'acide nucléique à détecter, ou de l'étalon d'acide nucléique à co-amplifier, ou lui est homologue.

11. Procédé selon au moins l'une des revendications 1 à 10, caractérisé en ce que la molécule d'acide nucléique couplée au colorant fluorescent est ajoutée au mélange réactionnel après que l'amplification soit terminée, auquel cas l'hybridation avec les acides nucléiques amplifiés s'effectue dans le cadre d'une étape de dénaturation thermique, suivie d'une étape de renaturation.

12. Procédé selon au moins l'une des revendications 1 à 4 et 10, caractérisé en ce que la molécule d'acide nucléique couplée au colorant fluorescent est ajoutée au mélange réactionnel avant la fin de l'amplification, la sonde étant ajoutée sous forme d'un ARN double brin non-amplifiable, ou sous forme d'un acide nucléique non-amplifiable et chimiquement modifié.

13. Procédé selon au moins l'une des revendications 1 à 12, caractérisé en ce qu'on utilise pour l'amplification au moins une amorce d'une paire d'amorces qui en position 5'-terminale code une région riche en G:C, constituée de préférence de 15 à 20 résidus G:C.

14. Procédé selon au mois l'une des revendications 1 à 10, caractérisé en ce que la sonde utilisée pour le dosage est un oligonucléotide ou un polynucléotide, qui comporte au moins deux éléments structuraux chimiques, qui sont à même d'absorber les ondes électromagnétiques et/ou de les émettre en raison d'une excitation, et où l'un des éléments structuraux peut, sous l'influence d'ondes électromagnétiques, créer ou défaire une liaison durable avec une autre position de l'oligonucléotide ou du polynucléotide.

15. Procédé selon la revendication 14, dans lequel l'élément structural qui absorbe et/ou émet une onde électromagnétique comporte un système chromophore.

16. Procédé selon la revendication 15, dans lequel le système chromophore provoque la luminescence d'un substituant de colorant.

17. Procédé selon au moins l'une des revendications 14 à 16, dans lequel l'élément structural qui crée ou détache des liaisons permanentes sous l'effet d'ondes électromagnétiques est un agent de réticulation photochimique.

18. Procédé selon la revendication 17, dans lequel l'agent de réticulation photochimique est un dérivé du psoralène.

19. Procédé selon au moins l'une des revendications 14 à 18, dans lequel la distance entre les éléments structuraux ne doit pas être inférieure à 8 à 12 et de préférence à 10 positions de nucléotides.

20. Procédé selon au moins l'une des revendications 1 à 19, caractérisé en ce qu'on utilise des systèmes de feuilles avec des renfoncements ou des creux servant d'espaces de réaction, qui peuvent être soudés par soudage thermique et qui servent de logements pour les mélanges de réactifs prêts à l'emploi sous forme lyophilisée ou fixée à une matrice, et qui conviennent aussi à une mesure optique directe.

21. Procédé selon au moins l'une des revendications 1 à 20, caractérisé en ce que les réactifs en place sont logés dans des matrices spatialement séparées, et, après la fermeture d'un espace de réaction peuvent, séparément dans le temps, être soumis au processus de réaction.

22. Procédé selon au moins l'une des revendications 1 à 21, caractérisé en ce que la distance entre les espaces de réaction est choisie de façon à permettre une évaluation avec des appareils du commerce, tels que les appareils formatés en microtitrage.

23. Procédé selon au moins l'une des revendications 1 à 22, caractérisé en ce que le mélange d'acides nucléiques est, pour analyse, soumis à un gradient de température à programmation dans le temps, de préférence linéaire, la variation du paramètre spectroscopique étant suivie en fonction du temps et/ou de la température.

24. Procédé selon la revendication 23, dans lequel l'analyse de la conformation du mélange d'acides nucléiques est réalisée à l'aide d'une électrophorèse sur gel à haute température, d'un procédé chromatographique, ou directement en solution homogène, ou dans une combinaison de ces procédés.

25. Procédé selon la revendication 23 et/ou 24, caractérisé en ce que, à partir de la dépendance entre le paramètre optique et la température et/ou le temps, il est possible de tirer des conclusions sur la présence et/ou le nombre et/ou l'homologie entre un acide nucléique recherché et l'étalon correspondant.

26. Procédé selon au moins l'une des revendications 1 à 25, caractérisé en ce que l'évaluation des données s'effectue en ligne à l'aide d'un système de traitement de données.

27. Procédé selon au moins l'une des revendications 1 à 26, dans lequel on utilise un système de compartiments de réaction, disposés selon la configuration d'une plaque de microtitrage, de préférence un système de feuille avec des réactifs prêts à l'emploi sous forme lyophilisée.

28. Procédé selon la revendication 27, caractérisé en ce que les réactifs sont fixés et/ou logés dans au moins une matrice hydrosoluble.

29. Procédé selon la revendication 27 et/ou 28, dans laquelle la matrice contient des stabilisants, de préférence le sucre, en particulier le tréhalose ou le saccharose.

30. Procédé selon au moins l'une des revendications 27 à 29, dans lequel le compartiment de réaction et/ou d'autres réservoirs de réactifs contiennent des amorces d'amplification, des constituants tampon, au moins une polymérase et des cofacteurs.

31. Procédé selon l'une des revendications 27 à 30, dans lequel la feuille qui obture les compartiments de réaction contient dans une matrice au moins un réservoir supplémentaire de réactifs, la sonde marquée y étant de préférence logée, avec les réactifs nécessaires à l'hybridation.

32. Utilisation d'une sonde oligo- ou polynucléotidique, comportant au moins un élément structural chimique, à même d'interagir avec des ondes électromagnétiques, en défaisant ou en créant des liaisons permanentes et/ou par absorption ou émission d'un rayonnement, cet élément structural ne représentant pas un substituant purine ou pyrimidine des composants nucléotidiques naturels, pour mettre en oeuvre le procédé selon au moins l'une des revendications 1 à 31.

33. Utilisation selon la revendication 32, dans laquelle l'élément structural pouvant créer des liaisons permanentes est le psoralène ou un dérivé du psoralène.

34. Utilisation selon l'une des revendications 32 et/ou 33, dans laquelle au moins l'un des éléments structuraux pouvant interagir avec un rayonnement électromagnétique est luminescent.

35. Utilisation selon au moins l'une des revendications 32 à 34, dans laquelle la distance entre les éléments structuraux de l'oligo- ou du polynucléotide est d'au moins 8 à 12 nucléotides.
